# EUROPEAN PATENT SPECIFICATION

(11) **EP 0 711 169 B1**
(45) Date of publication and mention of the grant of the patent: **10.12.2003**
(21) Application number: 93923753.3
(22) Date of filing: 28.09.1993
(51) Int. Cl.: A61K 31/56, A61K 31/565, A61P 25/22, A61P 25/24, A61P 25/18

(54) **ANDROSTANE STEROIDS AS NEUROCHEMICAL INITIATORS OF CHANGE IN HUMAN HYPOTHALAMIC FUNCTION AND RELATED PHARMACEUTICAL COMPOSITIONS AND METHODS**
ANDROSTANSTEROIDE ALS NEUROCHEMISCHE INITIATOREN EINER VERÄNDERUNG DER HYPOTHALAMUSFUNKTION BEIM MENSCHEN UND VERWANDTE PHARMAZEUTISCHE PRÄPARATE UND VERFAHREN
STEROIDES D'ANDROSTANE UTILISES EN TANT QU'INITIATEURS NEUROCHIMIQUES DE MODIFICATION DE LA FONCTION HYPOTHALAMIQUE HUMAINE, COMPOSITIONS PHARMACEUTIQUES ET PROCEDES ASSOCIES

(30) Priority: 15.06.1993 US 77359
(43) Date of publication of application: 15.05.1996
(60) Divisional application: 03076439.3
(73) Proprietor: PHERIN CORPORATION, Menlo Park, CA 94025 (US)
(72) Inventor: BERLINER, David L., Atherton, CA 94027 (US); ADAMS, Nathan William, Salt Lake City, UT 84123 (US); JENNINGS-WHITE, Clive L., Salt Lake City, UT 84109 (US)
(74) Representative: Beresford, Keith Denis Lewis
(86) International application number: US9309349
(87) International publication number: WO94028904

(56) References cited:
- WO-A-93/03732
- WO-A-93/10141
- GB-A- 1 175 219
- US-A- 3 908 007
- US-A- 4 330 538
- US-A- 5 272 134
- MONTI-BLOCH, L. ET AL: "Effect of putative pheromones on the electrical activity of the human vomeronasal organ and olfactory epithelium" THE JOURNAL OF STEROID AND MOLECULAR BIOLOGY, vol. 39, no. 4B, October 1991 (1991-10), pages 573-582, XP000862870
- REED, H.C.B. ET AL: "Androgen steroids as an aid to the detection of oestrus in pig artificial insemination" THE BRITISH VETERINARY JOURNAL, vol. 130, no. 1, 1974, pages 61-67, XP000863081
- ADAMCZYK, A. ET AL: "A new procedure for one-carbon homologation of ketones to alpha-hydroxy aldehydes" THE JOURNAL OF ORGANIC CHEMISTRY, vol. 49, 1984, pages 1378-1382, XP002034005
- TEMPLETON, J. F. ET AL: "Proton magnetic resonance spectra of 17-hydroxy-17-methyl-5-androstane C-3 ketone and C-3 alcohol isomers in chloroform-d and pyridine-d5" STEROIDS, vol. 41, no. 4, 1983, pages 485-91, XP002034006
- GHERA, E. ET AL: "Zinc-induced free-radical reactions of aromatic alpha-halogenoketones with terminal olefins" J. C. S. CHEM. COMM., November 1973 (1973-11), pages 858-859, XP002034007
- MACDONALD, B.S. ET AL: "The identification of 17-alpha-hydroxy-17-methyl-1,4- androstadien-3-one as a metabolite of the anabolic steroid drug 17-beta-hydroxy- 17-methyl-1,4-androstadien-3-one in man" STEROIDS, vol. 18, no. 6, 1971, pages 753-766, XP002034008
- CHEMICAL ABSTRACTS, vol. 62, no. 10, 10 May 1965 (1965-05-10) Columbus, Ohio, US; abstract no. 11871e, BARNIKOL-OETTLER, K. ET AL: "Wittig reaction of steroid hormones" XP002034010 & J. PRAKT. CHEM., vol. 27, no. 1-2, 1965, pages 18-33,
- ORTAR, G. ET AL: "Oxidation of 17-methylene steroids by thallium(III) and mercury(II) acetates" STEROIDS, vol. 27, no. 2, 1976, pages 197-203, XP002034009
- RUZICKA, L. ET AL: "Untersuchungen über den Zusammenhang zwischen Konstitution und Geruch bei Steroiden. Methyl-androstan- und Allo-pregnan-Derivate" HELVETICA CHIMICA ACTA, vol. 30, no. 3, 1947, pages 867-878, XP002068611
- Ohloff, G., et al., 'Structural and configurational dependence of the sensory process in steroids, Helv. Chim. Acta, 66(1), 1983, 192-217

## Description

### BACKGROUND

### Technical Field

This invention relates generally to pharmaceutical compositions for effectuating change in human hypothalamic function, thereby altering certain behavior and physiology mediated by the hypothalamus of individuals. More particularly, the invention relates to the use of certain Androstene steroids as neurochemical effectuators of physiology and behavior.

### Description of the Related Art

The present invention relates to certain compounds, namely Androstane steroids, particularly Androstene steroids, and use of these compounds in the preparation of a medicament suitable for nasal administration as human semiochemicals in order to alter hypothalamic function, thereby affecting certain consequent behavior and physiology, e.g., the reduction of anxiety. Androstane steroids are typified by testosterone and they are characterized by a four ring steroidal structure, a methylation at the 13-position and at the 10-position. Androstenes are a subset of Androstanes and have at least one double bond. Ohloff, G. et al. (Helv. Chim. Acta (1983) 66:192-217)
have shown that several members of this group of steroids have an odor which varies with different isomeric, diastereomeric, and enantiomeric forms. Some members of this group have been reported to act as a pheromone in some mammalian species - for instance, 5α-androst-16-en-3-one and 5α-androst-16-en-3α-ol in pigs (Melrose, D.R., et al., Br. vet. J. (1971) 127:497-502). These 16-Androstenes produced by the boar induce mating behavior in estrus sows (Claus, et al., Experimentia (1979) 35:1674-1675).

Some studies have noted that, in some species, various characteristics of certain 16-Androstenes (including 5a-Androst-16-en-3α-ol and 5α-Androst-16-en-3-one), such as concentration, metabolism, and localization, are sexually dimorphic (Brooksbank et al., J. Endocr. (1972) 52: 239-251; Claus, et al., J. Endocr. (1976) 68:483-484; Kwan, et al., Med. Sci. Res. (1987) 15:1443-1444). For instance, 5α-Androst-16-en-3α-ol and 5α-Androst-16-en-3-one, as well as Androsta-4,16-dien-3-one, have been found at different concentrations in the peripheral blood, saliva and axillary secretions of men and of women (Kwan, T.K., et al., Med. Sci. Res. (1987) 15:1443-1444), and their function as a human pheromone, to the extent of affecting choice and judgement, has been suggested (Id.; see also Gower, et al., "The Significance of Odorous Steroids in Axillary odour", In, Perfumery, pp. 68-72, Van Toller and Dodd, Eds., Chapman and Hall, 1988); Kirk-Smith, D.A., et al., Res. Comm. Psychol. Psychiat. Behav. (1978) 3:379). Androstenol (5α-androst-16-en-3α-ol) has been claimed to exhibit a pheromone-like activity in a commercial men's cologne and women's perfume (Andron™ for men and Andron™ for women by Jövan). Japanese Kokai No. 2295916, refers to perfume compositions containing androstenol and/or its analogues. 5α-Androstadien-3β-ol (and perhaps the 3α-ol) has also been identified in human axillary secretion (Gower, et al., Supra at 57-60. On the other hand, there is little agreement in the literature as to whether or not any putative pheromone actually plays any role in the sexual or reproductive behavior of mammals, particularly of humans. See: Beauchamp, G.K., et al., " The Pheromone Concept in Mammalian Chemical Communication: A Critique", In: Mammalian Olfaction, Reproductive Processes and Behavior, Doty, R.L., Ed., Academic Press, 1976). See also: Gower, et al., supra at 68-73.

An embodiment of the subject invention concerns the non-systemic, nasal administration of certain Androstene steroids to affect a specific behavioral or physiological response in human subjects, e.g., a reduction of negative affect, mood, and character traits. In particular, nasal administration provides for contacting neurochemical receptors of a heretofore poorly understood neuroendocrine structure, commonly known as the vomeronasal organ ("VNO"; also known as "Jacobson's organ"), with one or more steroid(s) or with compositions containing the steroid(s). This organ is accessed through the nostrils of most higher animals - from snakes to humans, and has been associated, inter alia, with pheromone reception in certain species (see generally Muller-Schwarze & Silverstein, Chemical Signals, Plenum Press, New York (1980)). The axons of the neuroepithelia of the vomeronasal organ, located supra palatinal, form the vomeronasal nerve and have direct synaptic connection to the accessory olfactory bulb and indirect input from there to the cortico-medial amygdaloid basal forebrain and hypothalamic nuclei of the brain. The distal axons of terminalis nerve neurons may also serve as neurochemical receptors in the VNO. Stensaas, L.J., et al., J. Steroid Biochem. and Molec. Biol. (1991) 39:553. This nerve has direct synaptic connection with the hypothalamus.

Johnson, A. et al. (J. Otolaryngology (1985) 14:71-79) report evidence for the presence of the vomeronasal organ in most adult humans, but conclude that the organ is probably non-functional. Contravening results which suggest that the VNO is a functional chemosensory receptor are reported by Stensaas, L., et al., supra; and by Moran, D.T., et al., Garcia-Velasco, J. and M. Mondragon; Monti-Bloch, L. and B. Grosser all in J. Steroid Biochem. and Molec. Biol. (1991) 39.

It is apparent that it would be desirable to identify and synthesize human semiochemicals and pheromones and to develop pharmaceutical compositions and methods of use to influence hypothalamic function. This invention relates to the unexpected discovery that, when nasally administered to human subjects, certain neurochemical ligands, particularly Androstene steroids, or pharmaceutical compositions containing Androstenes, specifically bind to chemoreceptors of certain nasal neuroepithelial cells and this binding generates a series of neurophysiological responses resulting in an alteration of hypothalamic function of an individual. When properly administered, the effect of certain of these compounds on the hypothalamus affects the function of the autonomic nervous system and a variety of behavioral-or physiological phenomena which include, but are not limited to the following: anxiety, premenstrual stress, fear, aggression, hunger, blood pressure, and other behavioral and physiological functions normally regulated by the hypothalamus. Otto Appenzeller. The Autonomic Nervous System. An introduction of basic and clinical concepts (1990); Korner, P.I. Central nervous control of autonomic cardiovascular function, and Levy, N.M. and Martin, P.J. Neural control of the heart, both in Handbook of Physiology; Section 2: Cardiovascular system - the heart, Vol I, Washington. DC, 1979, American Physiological Society; Fishman, A.P., et al. editors, Handbook of Physiology. Section 3: Respiratory System. Vol. II. Control of breathing. Bethesda MD. 1986. American Physiological Society.

In some instances a single Androstene steroid is administered, and in some instances combinations of Androstene steroids are administered.

### SUMMARY OF THE INVENTION

Accordingly, it is an object of this invention to provide pharmaceutical compositions which contain human semiochemicals or pheromones and are suitable for nasal administration in an individual.

It is also an object of this invention to use human semiochemicals or pheromones in the preparation of a medicament for altering hypothalamic function of an individual.

It is a further object of this invention to use human semiochemicals or pheromones in the preparation of a medicament for affecting physiological and behavioral functions of individuals which are normally regulated by the hypothalamus.

Finally, it is an object of this invention to provide means for altering hypothalamic function which have the following advantages: 1) administration directly to the chemoreceptors in the nasal passage and the vomeronasal organ, without pills or needles - i.e., non-invasively; 2) a mode of drug action through the nervous system and not through the circulatory system - thus brain function can be affected without consideration of the blood-brain barrier; 3) a direct means of affecting the hypothalamus - there is only one synaptic junction between pheromone receptors'and the hypothalamus; and, 4) providing a highly specific drug effect, thereby greatly reducing the potential for undesirable side-effects - this because sensory nerves are addressed to a specific location in the brain.

Additional objects, advantages and novel features of the invention will be set forth in part in the description which follows, and in part will become apparent to those skilled in the art upon examination of the following, or may be learned by practice of the invention.

Objects of this invention are achieved by providing a pharmaceutical composition suitable for nasal administration in an individual, said composition comprising a therapeutically effective amount of an androstane steroid selected from androsta-4,16-dien-3β-ol add 17-methylene-androst-4-en-3α-ol, and a pharmaceutically acceptable carrier.

In a second aspect, the invention relates to use of androsta-4,16-dien-3β-ol, or 17-methylene-androst-4-en-3α-ol in the preparation of a medicament suitable for nasal administration for
(a) reducing premenstrual stress in women, or
(b) alleviating the symptoms of psychoses, depression or anxiety in an individual.

### BRIEF DESCRIPTION OF THE DRAWINGS

Figure 1 illustrates the synthesis of Androsta-4,16-dien-3-one, Androsta-4,16-dien-3α-ol, and Androsta-4,16-dien-3β-ol.
Figure 2 is a graphic representation of the electrophysiological effect on receptor potential of the localized administration of particular steroids to the vomeronasal organ of female subjects (2A) and to the olfactory epithelium (2C). Figure 2B is a graphic comparison of the effect of an Androstane on the VNO receptor potential of male and female subjects.
Figure 3 is a graphic representation of the electrophysiological effect of the localized administration of particular steroids to the vomeronasal organ of male (3A) and female (3B) subjects.
Figure 4 depicts changes in receptor potential of the VNO after exposure of 5 females to two different Androstanes.
Figure 5 depicts sexual dimorphism in local and autonomic responses to the stimulation of the VNO with vomeropherins. Various vomeropherins (200 fmoles) and the diluent control were administered to 30 male and 30 female subjects (ages 20 to 45 ) as described. Bars indicate the mean response of the population.
Figs. 5A & B: EVG responses were measured as described in male (A) and female (B) subjects.
Figs. 5C & D: Electrodermal activity was measured as described. Changes (measured in xΩ) in response due to delivery of vomeropherins to the VNO of each subject are shown in male (C) and female (D) subjects.
Figs. 5E & F: Alpha-cortical activity was measured as described. Changes in response due to delivery of vomeropherins to the VNO of male (E) and female (F) subjects.
Figs. 5G & H: Skin temperature (ST) wad measured as described. Changes in response due to delivery of vomeropherins to the VNO of each subject are shown in male (G) and female (H) subjects.

The Compounds in the graphs are:
A = 1, 3, 5(10),16-Estratetraen-3-yl acetate
B = Androsta-4,16-dien-3-one
C = 1,3,5(10),16-Estratetraen-3-ol
D = 3-Methoxy-Estra-1,3,5(10),16-tetraene
E = Androsta-4,16-dien-3α-ol
F = Androsta- 4,16-dien-3β-ol

Figure 6 depicts electro-olfactgrams of male and female subjects induced by stimulation of the OE with olfactants and vomeropherins A: 400 fmoles of the olfactants 1-carvone and cineole as well as 200 fmoles of the vomeropherins A, B, C, D and F; and the stereoisomer E were applied separately as one second pulses to the OE of 20 subjects (both male and female) and each EOG response was recorded as described. The olfactants as well as E and B produced significant (p<0.01) local response. B: 400 fmoles of the olfactants 1-carvone and cineole do not induce a significant EVG response when delivered to the VNO of male and female subjects.

### DETAILED DESCRIPTION OF THE INVENTION

### I. Definitions

An "affect" is a transient feeling state. Typical negative affects are feelings of nervousness, tenseness, shame, anxiousness, irritability, anger, rage, and the like. "Moods" are longer lasting feeling states such as guilt, sadness, hopelessness, worthlessness, remorsefulness, misery, unhappiness and the like. "Character traits" are more permanent aspects of an individual's personality. Typical negative character traits are sensitivity, regretfulness, blameworthiness, stubbornness, resentfulness, bitterness, timidness, laziness and the like.

"Androstane steroids" are aliphatic polycyclic hydrocarbons characterized by a four-ring steroidal structure with a methylation at the 10- and 13-positions. An Androstene is a subset of Androstanes commonly understood to mean that the compound has at least one double bond. Commonly, unless a compound is described as a gonane, it is understood that the compound has a 19- carbon group. Furthermore, all derivatives which have the structural characteristics described above are also referred to generically as Androstane steroids.

A "chemoreceptor" is a receptor molecule displayed on the surface of a "chemosensory" neuroepithelial cell which binds in a stereospecif ic fashion to a particular ligand or ligands. This specific binding initiates a signal transduction which initiates an afferent nerve impulse. Chemoreceptors are found, inter alia, in taste buds, olfactory epithelium and vomeronasal tissue.

"Estrene steroids", as the term is used herein, are aliphatic polycyclic hydrocarbons with a four-ring steroidal structure, at least one double bond in the A-ring, no methylation at the 10-position and an oxo, hydroxyl or hydroxyl derivative such as an alkoxy, ester, benzoate, cypionate, sulfate or glucuronide, at the 3-position. Derivatives which contain these structural characteristics are also referred to generically as Estrene steroids.

The following structure shows the four-ring steroidal structure common to Androstane and Estrene steroids. In describing the location of groups and substituents, the following numbering system will be employed:

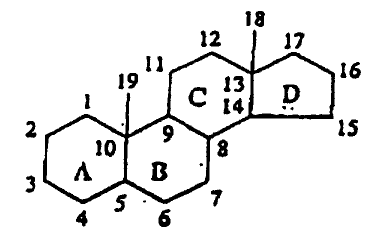

"Sexually dimorphic" refers to a difference in the effect of, or response to, a pharmaceutical agent between males and females of the same species.

An "effective amount" of a drug is a range of quantity and/or concentration which brings about a desired physiological and/or psychological effect when administered to an individual in need of the drug. In the present case, a needy individual is one with a physiological or behavioral trait which is normally regulated by the hypothalamus and wherein it is desirable to affect the function of the hypothalamus or the trait. The effective amount of a given drug may vary depending upon the function to be affected, the desired effect, route of administration, and the like. For example, when the steroid is administered as a solution applied to the facial skin of a subject an effective concentration is from 1 microgram/ml to 100 µg/ml, preferably 10 to 50 µg/ml and most preferably 20 to 30 µg/ml. When the steroid is introduced directly into the VNO an effective amount is about 1 picogram to about 1 nanogram, more preferably about 10 picograms to about 50 picograms. When the steroid is administered to the nasal passage, by ointment, cream or aerosol, or the like, an effective amount is about 100 pg to about 100 micrograms, preferably about 1 ng to about 10 micrograms. It follows that some drugs may be effective when administered by some routes, but not effective when administered by other routes.

The "hypothalamus" is the portion of the diencephalon comprising the ventral wall of the third ventricle below the hypothalamic sulcus and including structures forming the ventricle floor, including the optic chiasma, tuber cinereum, infundibulum, and mammillary bodies. The hypothalamus regulates the autonomic nervous system and controls several physiological and behavioral functions such as the so-called fight and flight responses, sexual motivation, water balance, sugar and fat metabolism, hunger, regulation of body temperature, endocrine secretions, and others. The hypothalamus is also the source of vasopressin which regulates blood pressure, and oxytocin which induces parturition and milk release. All hypothalamic functions are potentially modulatable by the semiochemical therapy described herein.

A "ligand", as used herein, is a molecule which acts as a chemical signal by specifically binding to a receptor molecule displayed on the surface of a receptor cell, thereby initiating a signal transduction across the cell surface. Binding of ligands to chemosensory receptors can be measured. Chemosensory tissue, such as vomeronasal neuroepithelium or olfactory neuroepithelium, contains a multiplicity of neuroreceptors cells, each displaying at least one cell surface receptor. Many of the receptor molecules have identical ligand specificity. Therefore, when the tissue is exposed to a ligand for which it has specificity (for example a exposure of the VNO to a semiochemical) a summated change in cell surface receptor potential can be measured.

As used herein, "lower alkyl" means a branched or unbranched saturated hydrocarbon chain of 1 to 4 carbons, such as, for example, methyl, ethyl, n-propyl, i-butyl and the like. "Alkoxy" as used herein is used in its conventional sense to mean the group -OR wherein R is alkyl as herein defined.

A "pheromone" is a substance that provides chemical means of communication between members of the same species through secretion and nasus reception. In mammals pheromones are usually detected by receptors in the vomeronasal organ of the nose. Commonly, pheromones effect development, reproduction and related behaviors. A "semiochemical" is a more general term which includes pheromones and describes a substance from any source which functions as a chemosensory messenger, binds to a specific neuroepithelial receptor, and induces a physiological or behavioral effect. A "vomeropherin" is a semiochemical whose physiologic effect is mediated through the vomeronasal organ.

A picogram (*p*g) is equal to .001 nanograms (ng). A ng is equal to .001 micrograms (µg). A µg is equal to .001 mg.

### II. Modes for Carrying Out the Invention

### A. Androstanes useful in the Invention

The invention is directed to certain Androstane steroids, namely androsta-4,16-dien-3β-ol and 17-methylene-androst-4-en-3α-ol.

Chart 1 includes the androstanes to which the invention is directed. The synthesis diagrams that follow depict intermediate and substructure syntheses for the preparation of some of the androstanes illustrated in the Chart:

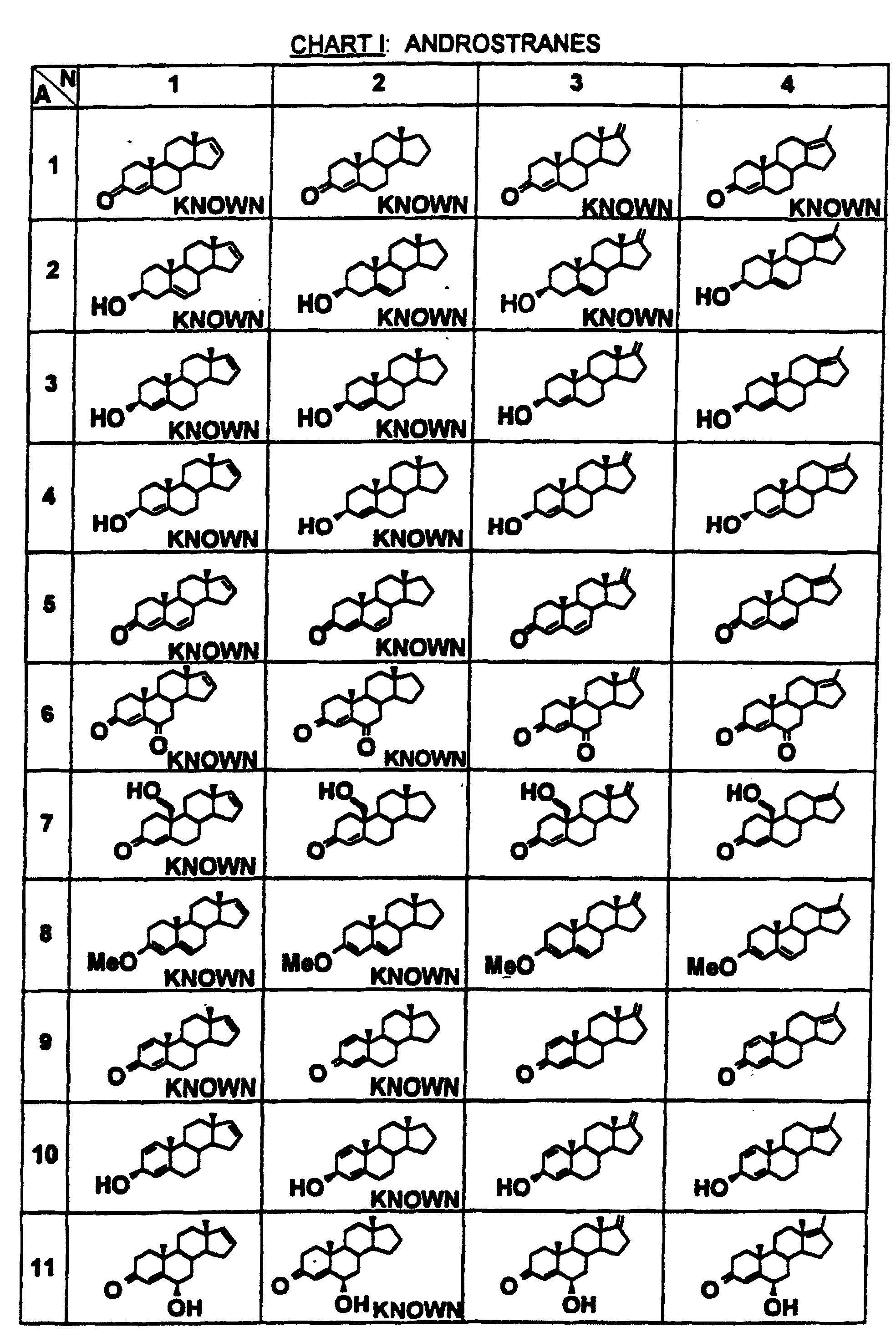

### SUBSTRUCTURE SYNTHESES

Referring to the preceding table, the following are exemplary syntheses for intermediates in a given row (A1-through A11) or column (N1 through N4).

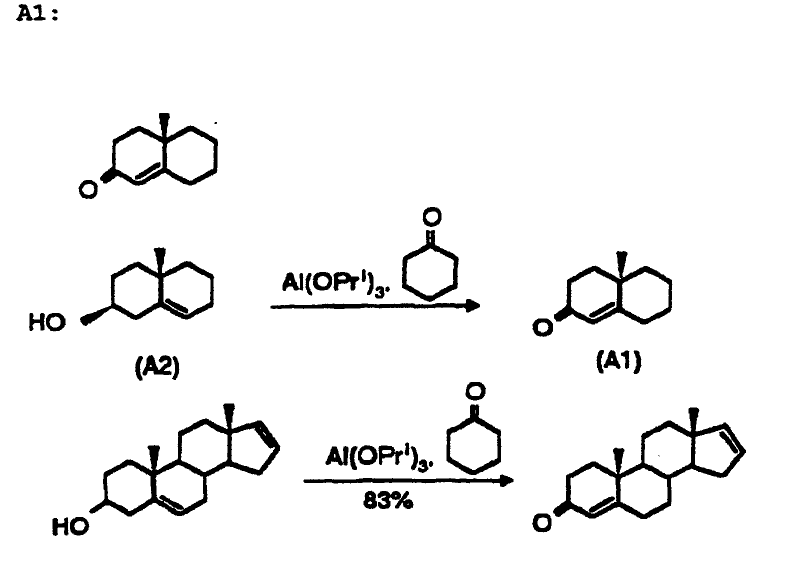

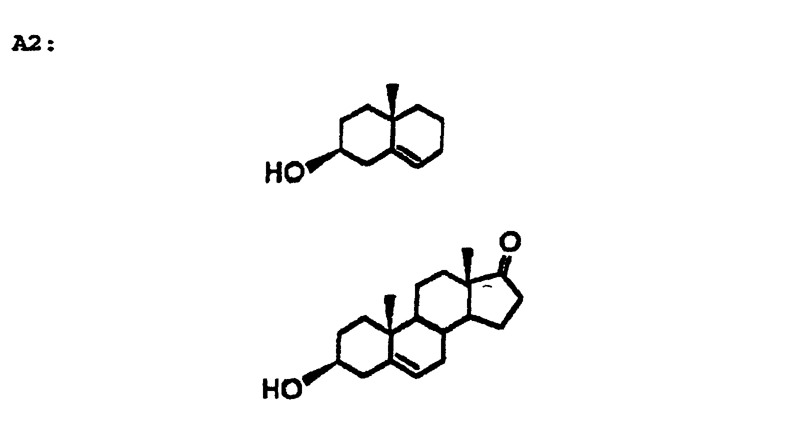

This is a commercially available substructure, for example, DEHYDRO EPI ANDROSTERONE.

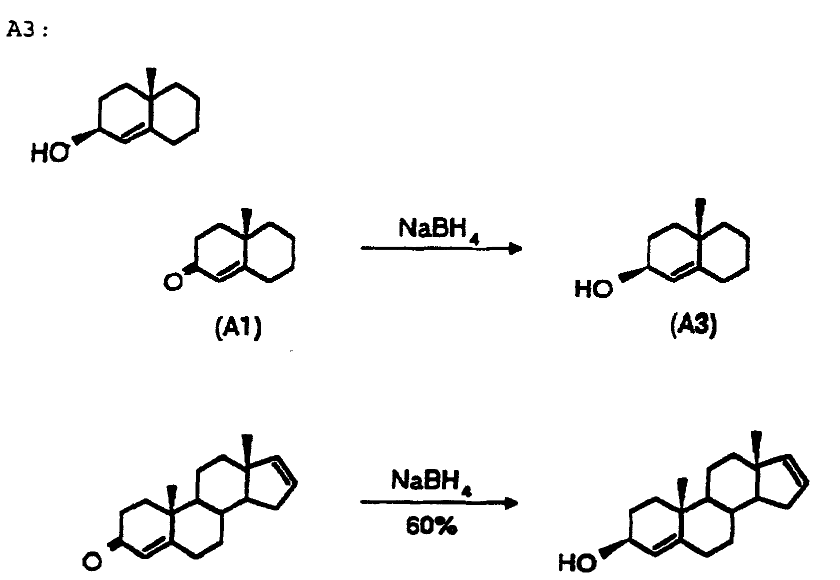

(Michio Matsui and David K. Fukushima, J. Org. Chem., 1970, Vol. 35, No. 3, p. 561-564).

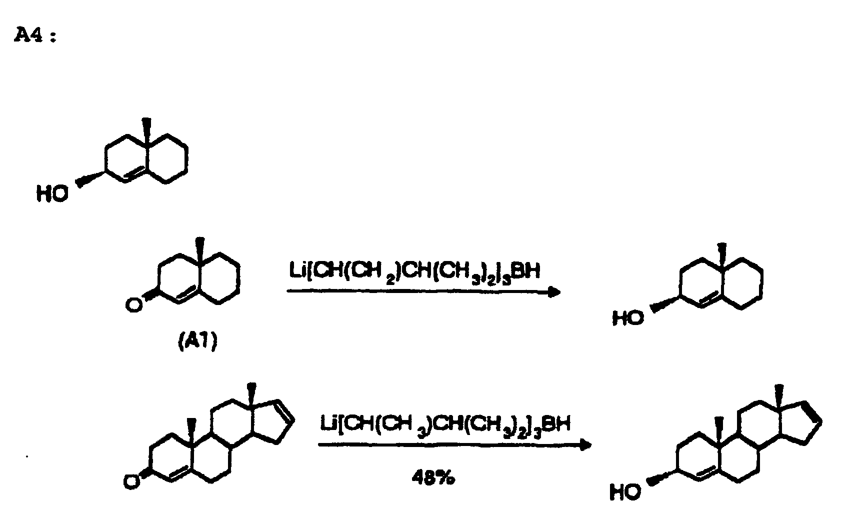

Ohloff, G. et al. (Helv. Chim. Acta (1983) 66: 192-217).

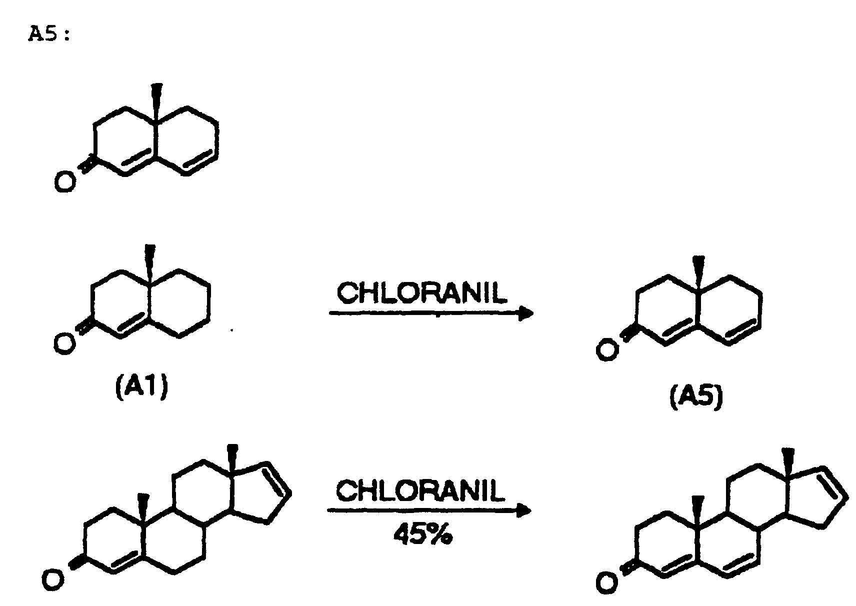

German Off. 2,631,915.

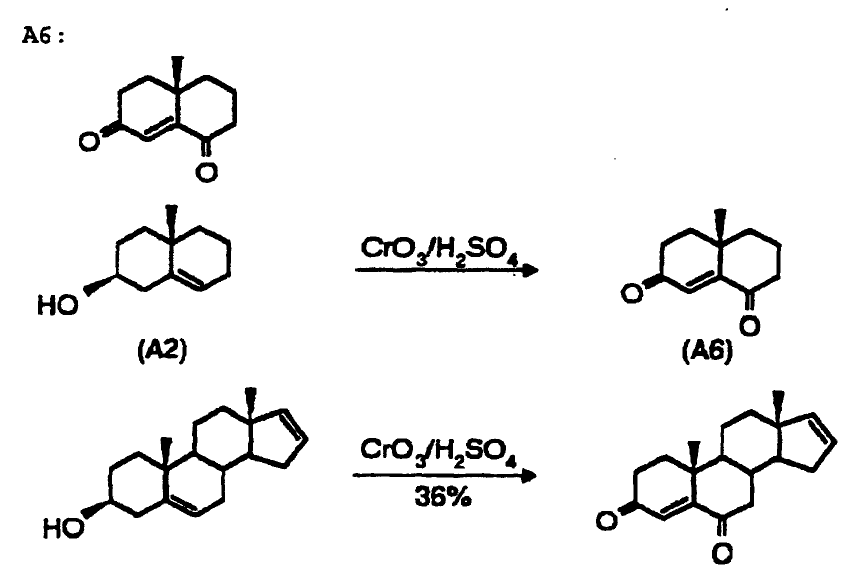

J. Römer, H. Wagner, and W. Sihade, Steroids, 1988, 51/5-6, p. 577-581).

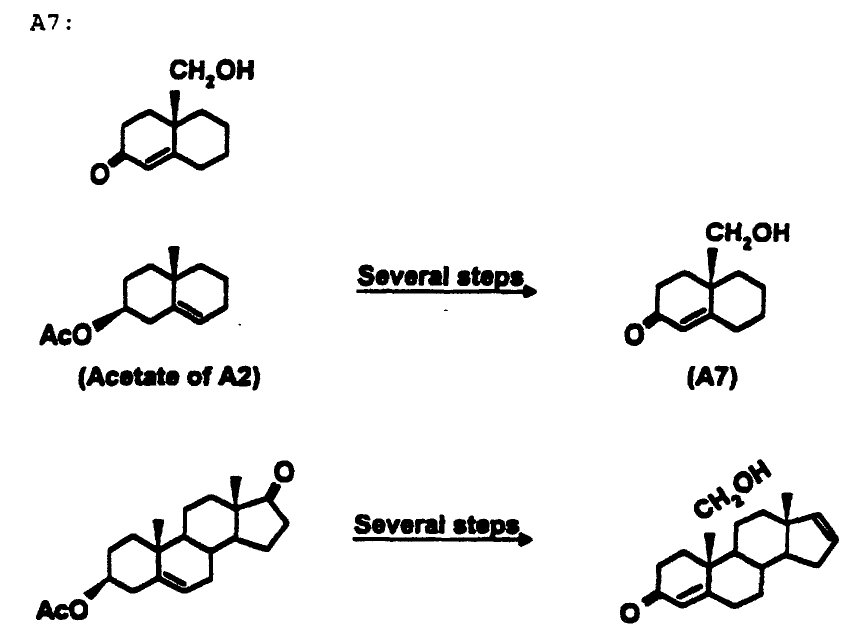

(Habermehl, et al., Z. Naturforsch. (1980) 256: 191-195).

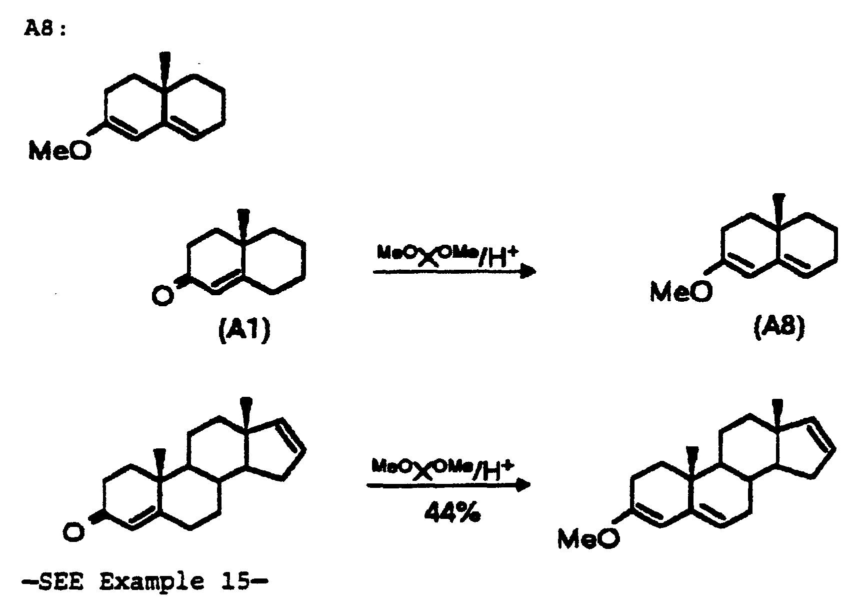

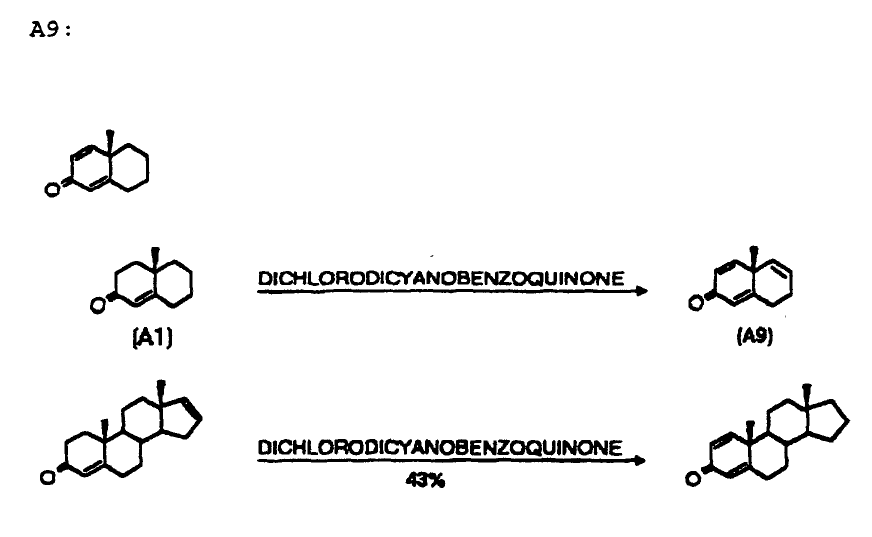

Ohloff, G. et al. (Helv. Chim. Acta (1983) 66: 192-217).

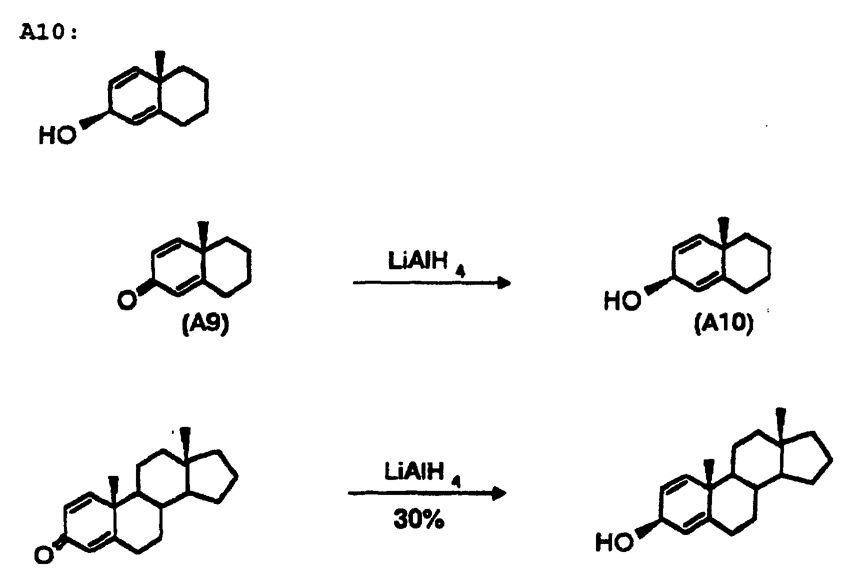

V. I. Mel'nikova and K. K. Pivnitskii, Zhurnal Organickeskoi Khisnii, 1972, Vol. 8, No. 1, pp. 68-74).

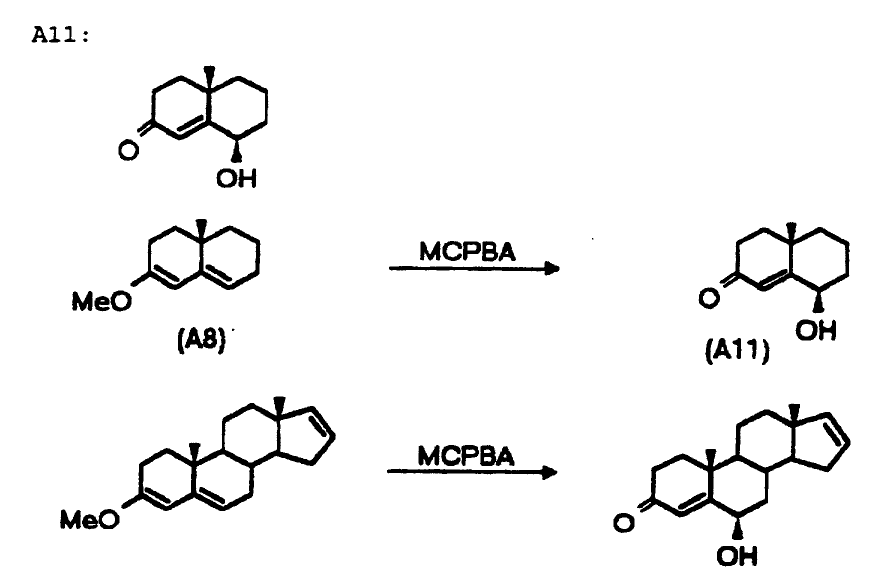

### - SEE EXAMPLE 19-

### Type N

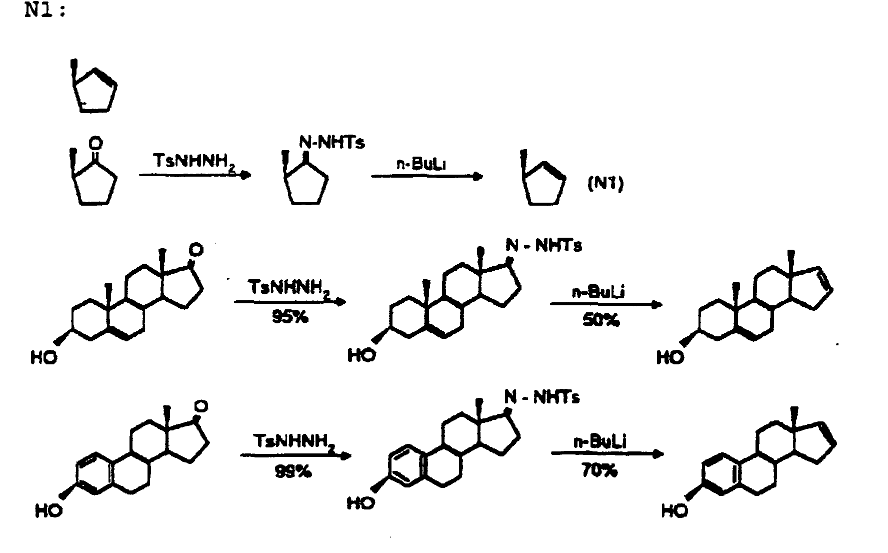

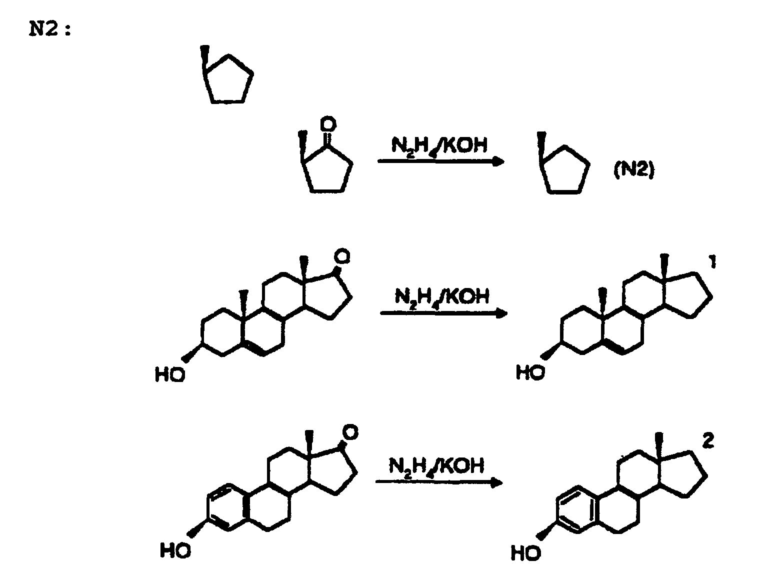

1. Robert H. Shapiro and Carl Djerassi, J. Am. Chem. Soc., 1964, 86, 2825.
2. Pilar Lup6n, Frances C. Canals, Arsenio Iglesias, Joan C. Ferrer, Albert Palomar, and Juan-Julio Bonet, J. Org. Chem. 1988, 53, 2193-2198.

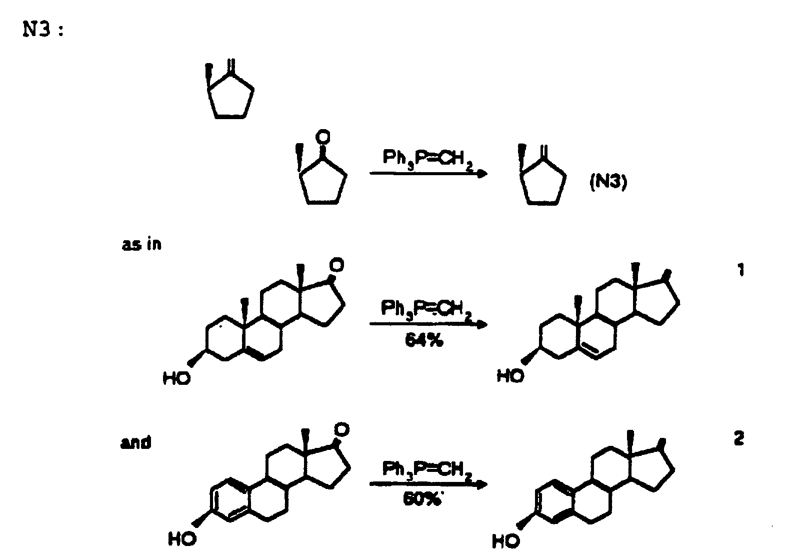

1. Günther Drefahl, Kurt Ponold and Hans Schick, Berichte, 1965, 98, 604.
2. Richard H. Peters, David F. Crows, Mitchell A. Avery, Wesley K. M. Chong, and Masako Tanabe, J. Med. Chem. , 1989, 32, 1642.

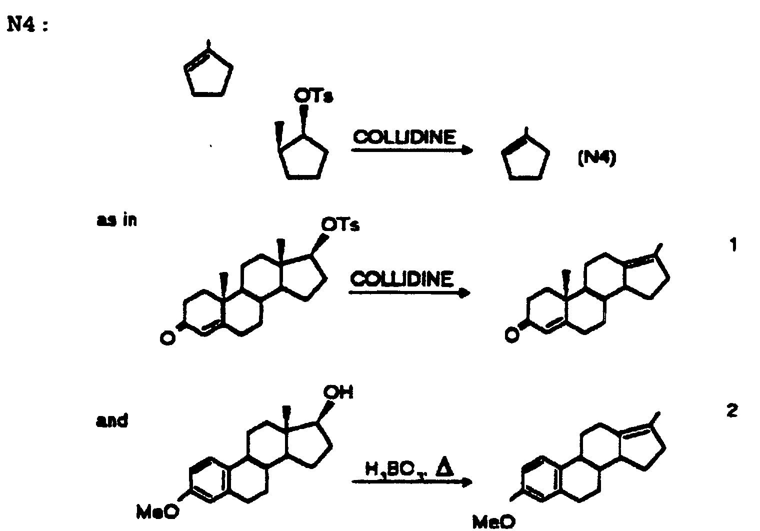

1. Franz Sondheimer, O. Mancera, M. Urquiza & G. Rosenkranz, J. Am. Chem Soc., 1955, 77, 4145.
2. William F. Johns, J. Org. Chem., 1961, 26, 4583.

### B. Synthetic Methods

### 1. Preparation of 3-, 18-and 19- position derivatives.

The compounds used in this invention are Androstane steroids substituted at the 3-, 18- and 19- positions.

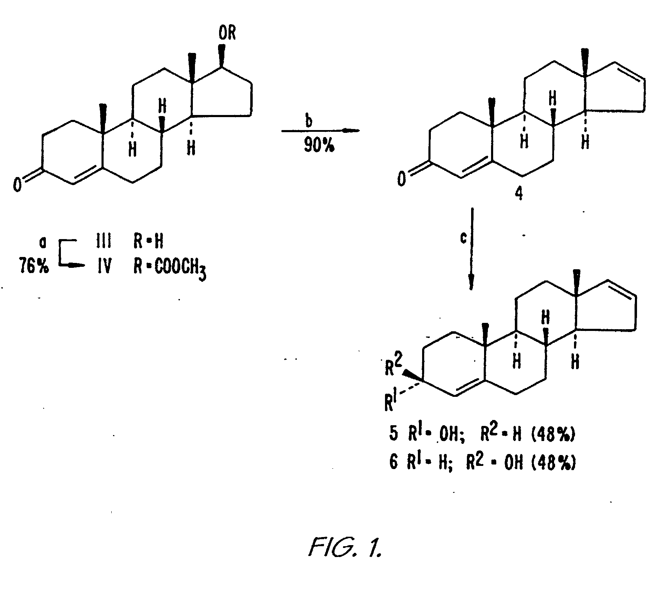

As shown in Figure 1, 17β-hydroxy-5α-Androstan-3-one (I) and methyl chloroformate (a) in pyridine gives the methyl carbonate, 17β-methoxycarbonyloxy-5α-Androstan-3-one (II) which provides a starting material for the 5α-Androst-16-en-(3-one and 3-ols) (Ohloff, supra at pg 200).

General procedures for synthetic reactions of steroids are known to those skilled in art. Where time and temperature of reactions must be determined, these can be determined by a routine methodology. After addition of the required reagents, the mixture is stirred under an inert atmosphere and aliquots are removed at hourly intervals. The aliquots are analyzed by chromatography to monitor the disappearance of starting material, at which point the work-up procedure is initiated. If the starting material is not consumed within twenty-four hours, the mixture is heated to reflux and hourly aliquots are analyzed, as before, until the starting material disappears. In this case the mixture is allowed to cool before the work-up procedure is initiated.

Purification of the products is accomplished by means of chromatography and/or crystallization, as known to those skilled in the art.

### C. Pharmaceutical Compositions and Methods of Use

The subject invention may be used in a method of altering the hypothalamic function of an individual. Another use is altering an autonomic function of an individual. These autonomic functions include but are not limited to heart rate, respiratory rate, brain wave patterns (percentage alpha cortical activity), body temperature. Other uses include, but are not limited to, methods of diminishing negative affect, negative mood or negative character traits of an individual. Another use is a method of treating female premenstrual stress. All of these uses are accomplished by means of the non-systemic, nasal administration of certain 16-Androstene steroids,and combinations thereof .

This particular mode of administration is distinguished from alternative modes, such as ingestion or injection, in several important ways, these by virtue of the direct contact with the VNO provided by the nasal administration of the steroid ligand. In the methods using this invention, the appropriate ligand is administered directly to the chemoreceptors in the nasal passage and the vomeronasal organ, without pills or needles - i.e., non-invasively. Drug action is mediated through binding of the ligands, described herein, to specific receptors displayed by neuroepithelial cells in the nose, preferably in the VNO. Furthermore, the mode of drug action is through the nervous system and not through the circulatory system - thus brain function can be affected without consideration of the blood-brain barrier. These methods of treatment provide a direct means of affecting the hypothalamus through the nervous system because there is only one synaptic junction between pheromone receptors and the hypothalamus. Because sensory nerves are addressed to a specific location in the brain, this method has a highly specific drug effect, thereby greatly reducing the potential of undesirable side-effects.

VNO contact is important because the VNO is associated with chemoreceptive/pheromonal function. The VNO consists of a pair of blind tubular diverticula which are found at the inferior margin of the nasal septum. The VNO contains neuro-epithelia, the axons of which have direct synapses to the amygdala and from there, to the hypothalamus. The existence of the VNO has been well documented in most terrestrial vertebrates including the human fetus; however, in adult humans it is generally thought to be rudimentary (See Johnson, et al., supra).

The ligand substances described herein, or their sulfated, cypionated, benzoated, propionated, or glucuronated derivatives, may be administered directly, but are preferably administered as compositions. They are prepared in a liquid dosage form such as, for example, liquids, suspensions or the like, preferably in unit dosage forms suitable for single administration of precise dosages. Liquid dosages may be administered as nose drops or as an aerosol. Alternatively, the active compound can be prepared as a creme or an ointment composition and applied topically within the nasal cavity. As another alternative, delivery may occur by controlled release of these agents by encapsulation either in bulk or at a microscopic level using synthetic polymers, such as silicone, and natural polymers such as gelatin and cellulose. The release rate can be controlled by proper choice of the polymeric system used to control the diffusion rate (Langer, R.S. and Peppas, N.A., Biomaterials 2,201, 1981). Natural polymers, such as gelatin and cellulose slowly dissolve in a matter of minutes to hours while silicone remains intact for a period of months. The compositions will include a conventional pharmaceutical carrier or excipient, and one or more of the active 16-Androstene compounds.

In addition, the compositions may include other medicinal agents, pharmaceutical agents, carriers, adjuvants, etc.

The most likely means of communication of a semiochemical ligand is the inhalation of a naturally occurring pheromone present on the skin of another. Several 16-Androstene steroids, including 5α-Androst-16-en-3α-ol and 5α-Androst-16-en-3-one, 4, 16-Androstadien-3-one, 5α-Androstadien-3β-ol, and perhaps 5α-Androstadien-3α-ol, are naturally occurring in humans and may be present on the skin. It is estimated that the naturally occurring maximum concentration of a 16-Androstene steroid on human skin is from 2 to 7 ng/cm². During intimate contact it is estimated that a human would be exposed to no more than 700 ng of a naturally occurring steroid. Since these compounds are relatively nonvolatile, it is estimated that, even during intimate contact, a human subject would inhale no more than 0.7 pg of a naturally occurring steroid from the skin of another. From the amount inhaled only about 1% would reach the receptors of the vomeronasal organ. Thus the estimated maximum natural exposure to naturally produced pheromones would be 0.007 pg.

The amount of semiochemical ligand administered will of course, be dependent on the subject being treated, the severity of the affliction, the manner of administration, the frequency of administration, and the judgment of the prescribing physician. However, a single dosage of at least about 10 picograms, delivered directly into the lumen of the vomeronasal organ, is effective in eliciting a transient autonomic response. When administered to the nasal cavity, the dosage is about 100 picograms to about 100 micrograms, preferably about 1 nanogram to about 10 micrograms, more preferably about 10 nanograms to 1 about microgram. The frequency of administration is desirably in the range of an hourly dose to a monthly dose, preferably from 8 times/day to once every other day, more preferably 1 to 3 times per day. ointments containing one or more active compounds and optional pharmaceutical adjuvants in a carrier, such as, for example, water, saline, aqueous dextrose, glycerol, ethanol, and the like, can be prepared using a base such as, for example, petroleum jelly, lard, or lanolin.

Liquified pharmaceutically administrable compositions can, for example, be prepared by dissolving, dispersing, etc. an active compound as defined above and optional pharmaceutical adjuvants in a carrier, such as, for example, water, saline, aqueous dextrose, glycerol, ethanol, and the like, to thereby form a solution or suspension. If desired, the pharmaceutical composition to be administered may also contain minor amounts of nontoxic auxiliary substances such as wetting or emulsifying agents, pH buffering agents and the like, for example, sodium acetate, sorbitan monolaurate, triethanolamine sodium acetate, triethanolamine oleate, etc. Actual methods of preparing such dosage forms are known, or will be apparent, to those skilled in this art; for example, see Remington's Pharmaceutical Sciences, Mack Publishing Co., Easton, PA, 15th Ed., 1975. The composition or formulation to be administered will, in any event, contain a quantity of one or more of the active compound(s) in an amount effective to alleviate the symptoms of the subject being treated.

For aerosol administration, the active ingredient is preferably supplied in finely divided form along with a surfactant and a propellant. Typical percentages of active ingredients are 0.001 to 2% by weight, preferably 0.004 to 0.10%.

Surfactants must, of course, be nontoxic, and preferably soluble in the propellant. Representative of such agents are the esters or partial esters of fatty acids containing from 6 to 22 carbon atoms, such as caproic, octanoic, lauric, palmitic, stearic, linoleic, olestearic and oleic acids with an aliphatic polyhydric alcohol or its cyclic anhydride such as, for example, ethylene glycol, glycerol, erythritol, arabitol, mannitol, sorbitol, and hexitol anhydrides derived from sorbitol (the sorbitan esters sold under the trademark "Spans") and the polyoxyethylene and polyoxypropylene derivatives of these esters. Mixed esters, such as mixed or natural glycerides, may be employed. The preferred surface-active agents are the oleates or sorbitan, e.g., those sold under the trademarks "Arlacel C" (sorbitan sesquioleate), "Span 80" (sorbitan monoleate) and "Span 85" (sorbitan trioleate). The surfactant may constitute 0.1-20% by weight of the composition, preferably 0.25-5%.

The balance of the composition is ordinarily propellant. Liquefied propellants are typically gases at ambient conditions, and are condensed under pressure. Among suitable liquefied propellants are the lower alkanes containing up to five carbons, such as butane and propane; fluorinated or fluorochlorinated alkanes, such as are sold under the trademark "Freon". Mixtures of the above may also be employed.

In producing the aerosol, a container equipped with a suitable valve is filled with the appropriate propellant, containing the finely divided active ingredient and surfactant. The ingredients are thus maintained at an elevated pressure until released by action of the valve.

Yet another means of administration is topical application of a volatile liquid composition to the skin, preferably facial skin, of an individual. The composition will usually contain an alcohol such as ethanol or isopropanol. A pleasant odorant may also be included in the composition.

### D. Measuring Affect, Mood and Character Trait.

Feeling states associated with affects, moods and character traits are generally measured by use of a questionnaire. For example questionnaires comprising a number of adjectives which refer to feeling states may be administered to an individual. The individual evaluates his or her feeling state described by the adjective and rates the intensity of the feeling on a numerical scale. Clustering of re lated adjectives and statistical analysis of a subject's evaluation of each adjective provides a basis for the measurement of various feeling states.

Alternatively, feeling states may be measured by autonomic changes, such as those used in polygraphic evaluations (galvanic skin response, pulse rate and the like). Cabanac, M. Annual Review of Physiology (1975) 37:415; Hardy, J.D., "Body Temperature Regulation", Chapter 59, pp. 1417. In: Medical Physiology. Vol. IIEd.: VB Mountcastle (1980); Wolfram Bouscein. Electrodermal Activity (Plenum Press 1992). In addition, non-verbal cues such as facial expression and body posture may be evaluated.

### E. Uses in the Treatment of Certain Types of Psychiatric Disorders.

Compositions suitable for topical and/or 10 intranasal administration, and which contain one or more of the steroidal substances described herein, are useful as therapeutics with efficacy in the treatment of certain types of psychiatric disorders, particularly certain types of neuroses. In a preferred embodiment, and unlike other drug formulations designed to invoke a systemic effect, the composition is formulated to minimize the likelihood of mucosal or transdermal absorption since the mode of action of these substances as therapeutics is by stimulation of neural receptors in the nose, more particularly in the VNO. Since the compositions of the instant invention are effective as the result of stimulation of chemosensory receptors in the VNO and not as a result of systemic circulation, absorption and the attendant incorporation of the active ingredient or ingredients into systemic circulation would not be efficacious, but rather might actually increase the likelihood of side effects. These compositions are useful when administered in a method such that one or more of the

16-Androstenes of the composition are provided to the VNO of the subject.

Treatable neuroses may be acute and/or transient or they may be persistent or recurrent. They generally involve abnormal symptoms that may include mood changes (anxiety, panic, depression) or limited abnormalities of thought (obsessions, irrational fears) or of behavior (rituals or compulsions, pseudoneurological or hysterical conversion signs). In such disorders, these compositions may have some beneficial effects for short periods, particularly by modifying associated anxiety or depression or the like.

Other characterological disorders may also be treatable by the intranasal administration of the compositions of this invention. These conditions include characteristic personality styles, e.g., paranoid, withdrawn, psychopathic, hypochondrial and the like; or behavioral patterns, e.g., abuse of alcohol or other substances, socially deviant or perverse behavior and the like that may run counter to societal expectations.

Certain physical or physiological conditions, both normal (such as menstruation) and disease or injury related (such as chronic illness) may have a psychological component that manifests as a psychiatric disorder, neurotic disorder, or as an anxious or depressed affect or mood. These conditions are also treatable by methods of administration of the compositions of the present invention such that one or more of the androstenes of the composition are provided to the VNO of the subject.

### i. Anxiety

Anxiety is a disagreeable emotional state characterized by feelings of uneasiness, impending danger, apprehension or tension. Compositions suitable for intranasal administration, and which contain one or more of the androstenes of the composition described herein, are useful as therapeutics with efficacy in the reduction of anxiety. There are numerous manifestations of this disorder ranging from mild perturbations to syndromes which can be incapacitating. Anxiety is not only a cardinal symptom of many psychiatric disorders but also a component of many normal physiological and social conditions. In addition, symptoms of anxiety are commonly associated with depression and especially with dysthymic disorder (neurotic depression) and many personality disorders. Anxiety disorders can be separated and include panic disorders, phobias and generalized anxiety disorders, post-traumatic stress disorder and obsessive-compulsive disorder.

### ii. Drugs used in the treatment of anxiety

Drug treatment is the major treatment method of anxiety, often used in conjunction with behavior therapy. The drugs used most frequently to treat anxiety are the benzodiazepines. Benzodiazepines have hypnotic, sedative, anxiolytic, anticonvulsant and muscle relaxant actions. Consequently, they are used for may indications and in many different conditions than anxiety disorders (e.g., insomnia, alcohol withdrawal states, muscle spasms, epilepsy, anesthesia and sedation for endoscopic procedures). These drugs are relatively safe, and have advantages in comparison with the previously used barbiturates. They have a rapid onset of action, and produces a sense of euphoria. However, despite their good safety record, benzodiazepines are associated with a number of properties that limit their use including sedation, ataxia, memory impairment, reduced motor coordination and dangerous addictive effects, especially when used with alcohol. Both physicians and patients are increasingly aware of this potential for addiction; and there is an evident desire, if not trend, to move away from their longterm use.

Recently, a new class of non-benzodiazepine anxiolytic agents, azaspirodecanediones, which may have more selective anxiolytic properties, has been approved. The first marketed member of this class is buspirone. This compound has been reported to be as effective as benzodiazepines in the treatment of Generalized Anxiety Disorder. Compared to benzodiazepines, buspirone is less sedating, potentiates the effect of ethanol to a lesser degree, and has a low addictive potential. However, in normal clinical use, it has been found to be difficult to substitute buspirone for benzodiazepines in patients already receiving benzodiazepines since buspirone does not create a feeling of euphoria, and patients do not feel as satisfied as when taking benzodiazepines. In addition, buspirone's slow onset of action (1-3 weeks) limits its effectiveness in treating acute anxiety. Systemically administered buspirone anxiolytic has been shown to have a high affinity for the CNS serotonin 5-HT receptor and this is thought to be the mode of anxiolytic action.

Another class of new anxiolytic is the systemically administered benzofuran derivatives. These substances demonstrate a high affinity for the CNS dopamine D2 receptor.

### iii. Mood (Affective) disorders

The compositions of the instant invention are also useful in the treatment of some of the symptoms of certain types of mood disorders. Mood disorders such as major depression and mania are characterized by changes in mood as the primary clinical manifestation. Either extreme of mood may be associated with psychosis, characterized by disordered delusional thinking and perceptions, often congruent with the predominant mood. Conversely, psychotic disorders may have associated or secondary changes in mood. Similarly, normal grief, sadness and disappointment and the dysphoria or demoralization often associated with medical illness or with normal cyclic dysfunctions such as premenstrual stress may also be mitigated by the intranasal application of the compositions of the instant invention.

### iv. The mode of action of the compositions of the instant invention

The mode of action of the compositions of the instant invention is quite different from any modes of treatment that have been heretofore reported. The active steroid and steroid-like compounds described in the instant invention stimulate and/or bind to receptors in the VNO that are directly available to topical administration. Direct access to CNS receptors is not required. Stimulation of these receptors then produces a signal transmitted through a neural pathway and inducing a neuropsychic response, likely in the hypothalamic region of the subject's brain. This response manifests as discrete changes in a variety of autonomic functions, including but not limited to pulse rate, respiratory frequency, E.E.G. patterns, evoked potential, skin temperature, galvanic skin response and pupillary diameter. The response also manifests as a measurable reduction of negative affect and negative mood.

While the relationship between the modification of hypothalamic function and the treatment of psychiatric disorders is not fully understood, it is clear that the compositions of this invention are effective in the reduction of self-evaluative negativity - negative mood characteristics as well as negative character. These changes are accompanied by autonomic changes, effected by the hypothalamus, that are consistent with an increase in parasympathetic tone (or alternatively a decrease in sympathetic tone).

Since the active compounds of this invention are pheromone-like in their specificity, the compounds exhibit a species specificity in their stimulation and activity add therefore demonstration of efficacy cannot be done in animal models.

### III. Examples

The following examples are intended to illustrate but not to limit the invention.

Abbreviations used in the examples are as follows: aq.= aqueous; RT.=room temperature; PE=petroleum ether (b.p. 50-70°); DMF=N,N-dimethylformamide; DMSO=dimethyl sulfoxide; THF=tetrahydrofuran.

### Example 1 - Androsta-4,16-dien-3α-ol (5) and -3β-ol (6).

These syntheses are depicted in Figure 1. Androsta-4,16-dien-3-one (4) was reduced at -55° with lithium tris (1,2-dimethylpropyl) hydridoborate in THF (c) as described for the preparation of 2 (Figure 1). Chromatography on silica gel with CH₂Cl₂/ethyl acetate 9:1 gave pure axial alcohol 5 (48% yield) and pure equatorial alcohol 6 (48% yield). Analytical samples were further purified by recrystallization (from PE at -30° for 5, from cyclohexane at RT. for 6).

Data of 5. M.p. 77-79°, [a]_{D} +120.6° (C = 1.26) - IR. (CDCl₃): 3620m, 3440m br., 1660m, 1595w. ¹H-NMR, (360 MHz): 0.79 (s, 3 H); 1.02 (s, 3 H); 4.07 (m, w_{½} ≈ 10, 1 H); 5.48 (d x d, J 5 and 2, 1 H); 5.71 (m, 1 H); 5.85 (m, 1 H).

Data of 6. M.p. 116.1190, [a]_{D} +53.9° (C = 1.28) ([47): m.p. 116.1180, [⁸)D +59.3° (C = 0.4) - IR. (CDCl₃): 3610m, 3420m br., 3050m, 1660m, 1590w. - ¹H- NMR. (360 MHz): 0.78 (s, 3 H) ; 1.08 (s, 3 H); 4.15 (m, w_{½} ≈ 20, 1 H); 5.30 (m, w_{½} ≈ 5, 1 H); 5.71 (m, 1 H); 5.85 (m, 1 H).

### Example 2 - Synthesis of 17-methylene-Androst-4-en-ols.

To 20-homoandrosta-4,17-dien-3-one (119.0 mg, 0.4184 mmol) in 5 mL of methanol were added sodium borohydride (6.0 mg, 0.16 mmol) and 77 µL of water. After stirring 2 h further sodium borohydride (32.0 mg, 0.846 mmol) was added and the mixture was stirred overnight. After concentrating under reduced pressure the residue was purified by preparative TLC (5% ethyl acetate/hexane on silica gel) to give a more polar (59.8 mg) and a less polar (1.7 mg) product.

### Comparative Example 3 - Electrophysiology of Androstane Stimulation of the Human VNO and Olfactory Epithelium.

A non-invasive method has been employed to record local electrical potentials from the human vomeronasal organ (VNO) and from the olfactory epithelium (OE). Localized gaseous stimulation was applied to both nasal structures at different instances using specially designed catheter/electrodes connected to a multichannel drug delivery system. This electrode and delivery system has been described by Monti and Grosser (J. Steroid Biochem. and Molec. Biol. (1991) 39:573) and in commonly owned, copending USSN 07/771,414, incorporated herein by reference. The local response of the VNO and the OE showed a correlation with the concentration of the ligand stimulus.

The study was performed on ten clinically normal (screened) volunteers - 2 males and 8 females, ranging in age from 18 to 85 years. The studies were conducted without general or local anesthetics.

The catheter/electrodes were designed to deliver a localized stimulus and simultaneously record the response. In the case of VNO recording, the right nasal fosa of the subject was explored using a nasoscope (nasal specule) and the vomeronasal opening was localized close to the intersection of the anterior edge of the vomer and the nasal floor. The catheter/electrode was gently driven through the VNO-opening and the electrode tip placed in the organ's lumen at 1 to 3 mm from the opening. The nasoscope was then removed. In the case of the OE, recording the procedure was similar except the positioning of the catheter/electrode was gently placed deep in the lateral part of the medial nasal duct, reaching the olfactory mucosa.

Localized gaseous stimulation was done through the catheter/electrode. A constant stream of clean, nonodorous, humidified air at room temperature was continuously passed through a channel of the stimulating system. The stimulating ligand substances were diluted in propylene glycol, mixed with the humidified air, and puffed for from 1 to 2 seconds through the catheter/electrode. It is estimated that this administration provides about 25 pg of the steroid-ligand to the nasal cavity.

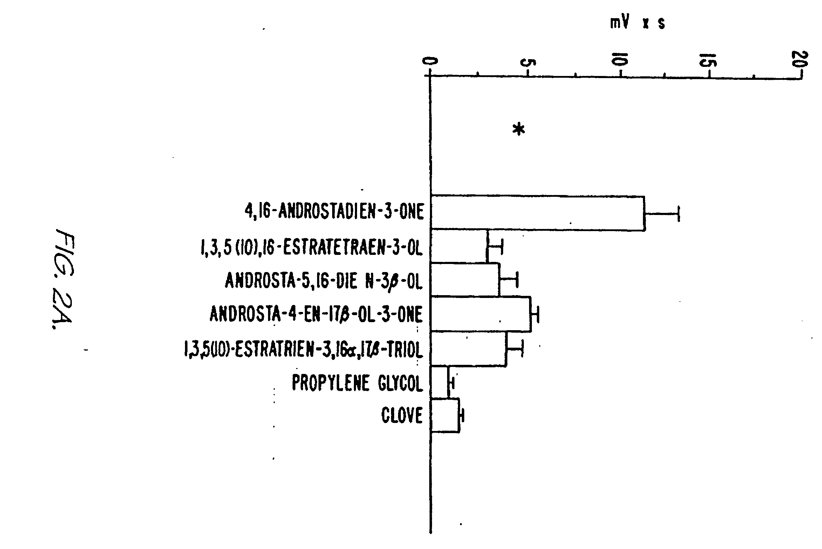

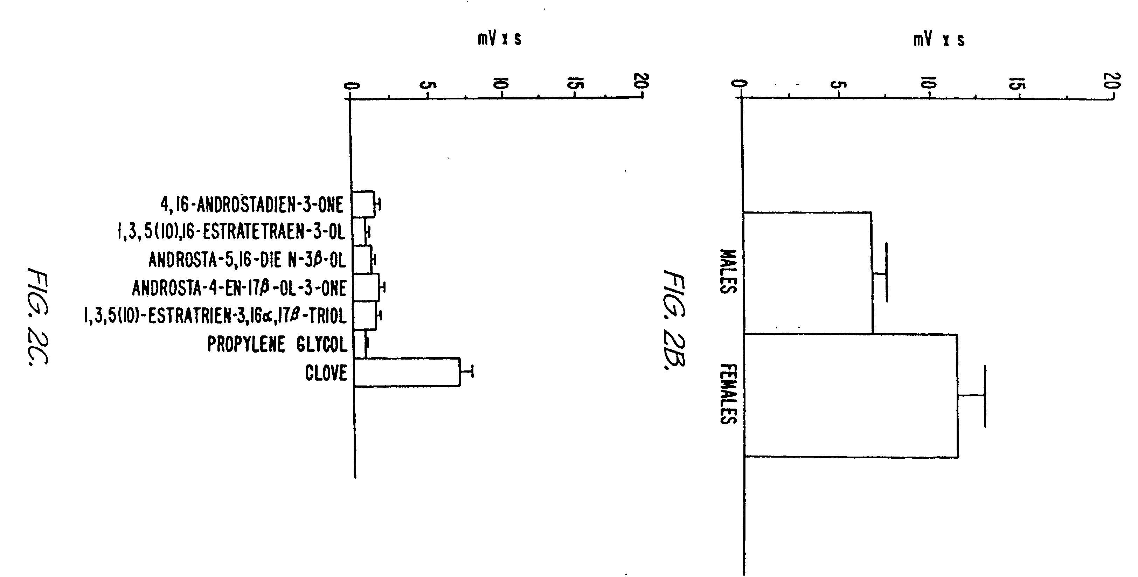

The results of this study are presented in Figures 2A, 2B and 2C. The response is measured in millivolt-seconds (mV x s). Androsta-4,16-dien-3-one elicits a significantly stronger VNO response in females than do the other compounds tested (Fig. 2A). Furthermore, the VNO response to Androsta-4,16-dien-3-one is sexually dimorphic - twice as strong in females as it is in males (Fig. 2 B). In contrast, the OE response in both males and females is low compared to a strong odorant such as clove (Fig. 2C).

### Example 4. - Measurements of the Change in Receptor Potential of the Neuroepitihelium of the VNO in Response to Various Steroids.

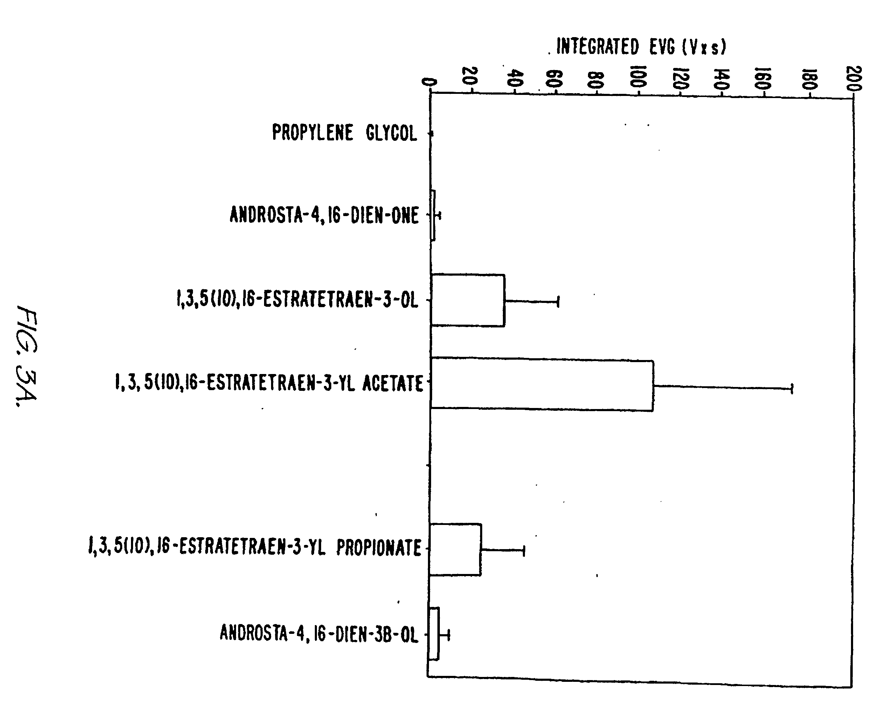

The change in receptor potential in response to five different ligands was measured in 40 female (Fig. 3A) and 40 male (Fig. 3B) subjects. Each subject was administered 60 pg of each of seven substances as indicated in the figure. The substances were administered separately for 1 second, using the procedure described in Comparative Example 3. The change in potential of the neuroepithelium of the VNO was recorded over time and the integral of the change in potential for each of the forty subjects was averaged. The results are shown in the figure. Comparison of Figures 3A and 3B show that each steroid is sexually dimorphic in its activity, and that some ligand substances are stronger in males while others are stronger in females.

### Example 5 - Comparison of the Chanae in Receptor Potential Induced by Two Androstane Steroids.

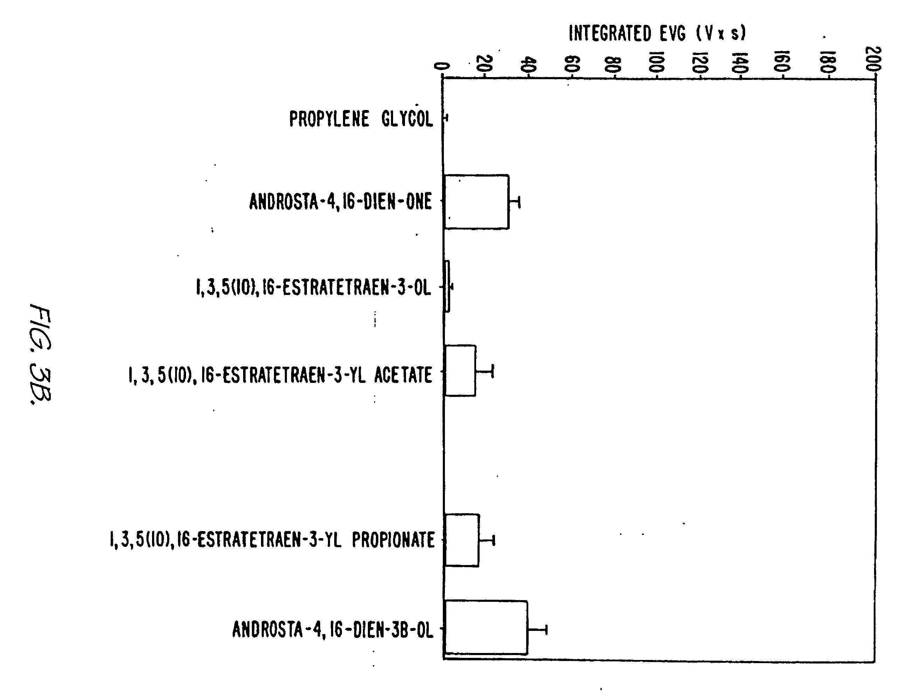

60 picograms of each ligand steroid and of a propylene glycol control were administered to 5 female subjects as described in Comparative Example 3, As shown in Figure 4, Androsta-4,16-dien-3β-ol induced a greater change in receptor potential than did Androsta-4,16-dien-3-one.

### Example 6 - Treatment of Women for Premenstrual Stress.

Women experiencing the symptoms of premenstrual stress (PMS) are provided with a pharmaceutical preparation of an Androstane steroid (preferably Androsta-4, 16-dien-3 β -ol) suitable for nasal administration. The steroid is provided as an ointment at a concentration of about 1 microgram/ml and about 0.1 ml is applied. The ointment is applied just inside the nare of each nostril, three times daily. A similar method of treating PMS uses an aerosol preparation of the same steroid. The aerosol is sprayed into each nostril threes times daily.

### Example 7 - Electrophysiological Studies

The following electrophysiological studies were performed in 60 clinically normal human volunteers of both sexes (30 male and 30 female) whose ages ranged from 20 to 45 years. No anesthetics were used, and female subjects were excluded if pregnant.

The stimulation and recording system consists of a "multifunctional miniprobe" described elsewhere (Monti-Bloch, L. and Grosser, B.l. (1991) "Effect of putative pheromones on the electrical activity of the human vomeronasal organ and olfactory epithelium," J. Steroid Biochem. Molec. Biol. 39:573-582.). The recording electrode is a 0.3 mm silver ball attached to a small (0.1 mm) silver wire insulated with Teflon© the surface of the electrode is first treated to produce a silver chloride interface, and is then covered with gelatin It is positioned within a small caliber Teflon© catheter (dia = 5 mm) such that the tip of the electrode protrudes approximately 2 mm. The Teflon© catheter is 10 cm in length and constitutes the terminal extension for a multichannel delivery system which delivers a continuous air stream carrying discreet pulses of chemosensory stimuli. The air stream first passes into a small chamber and is bubbled through a solution containing either a vomeropherin or an olfactant in a diluent or the diluent alone. A solenoid is used to rapidly redirect the air stream from the chamber to a route which bypasses the chamber. This creates a discreet pulse of stimulant in the air stream. A second, outer Teflon® tube with a diameter of 2 mm surrounds the catheter-electrode assemblage, and its central end is connected to an aspirator that provides continuous suction of 3ml/s. This concentric arrangement of the outer suction tube allows the emitted chemosensory stimuli to be localized to an area we call a "minifield" (approx. dia = 1 mm), and it avoids diffusion of substances either to the area outside the intended stimulation site or into the respiratory system. The entire stimulating and recording assemblage may be positioned either on the neurosensory epithelium within the VNO, or on the surface of the olfactory or respiratory epithelium.
Electro-vomeronasogram (EVG): Recordings are carried out in a quiet room with the subject supine; the multi-functional miniprobe is initially stabilized within the nasal cavity using a nasal retractor placed in the vestibule. Reference and ground electrodes consist of silver discs (8 mm), both of which are positioned on the glabella.

The entrance to the VNO, or vomeronasal pit, is identified by first dilating the nasal aperture and vestibule. A 6x magnifying binocular loupe with halogen illumination is then used to introduce the tip of the Teflon® catheter and recording electrode assemblage into the VNO opening where it is stabilized at an approximate depth of 1 mm within the vomeronasal passage. Optimal placement of the recording electrode is signaled after testing for an adequate depolarization in response to a test substance.

Electrical signals from the recording electrode are fed to a DC amplifier after which they are digitized, computer monitored, and stored. The peak-to-peak amplitude of the signals is measured, and the area under the depolarization wave is integrated, while continuously monitoring the signal both on the computer screen and on a digital oscilloscope. Artifacts produced by respiratory movements are deleted by training the subjects to practice mouth breathing with velopharyngeal closure. Chemosensory Stimulants: Olfactory test substances are cineole, and 1-carvone; vomeropherins are A, B, C, D, E and F. Samples of vomeropherins in concentration of 25-800 fmoles are delivered in the continuous air stream for durations from 300 milliseconds to 1 second. Usually, intervals of 3 to 5 minutes separated each series of short test pulses. All components of the lines carrying the test stimuli are made of Teflon©, glass or stainless steel and are carefully cleaned and sterilized before each use. Electro-olfactgram (EOG): Olfactory recordings employed the same stimulating and recording multifunctional miniprobe as that used for the VNO. The tip was slowly introduced until the recording electrode touched the olfactory mucosa. Adequate placement was signaled by a depolarization in response to a pulse of the odorant test substance.

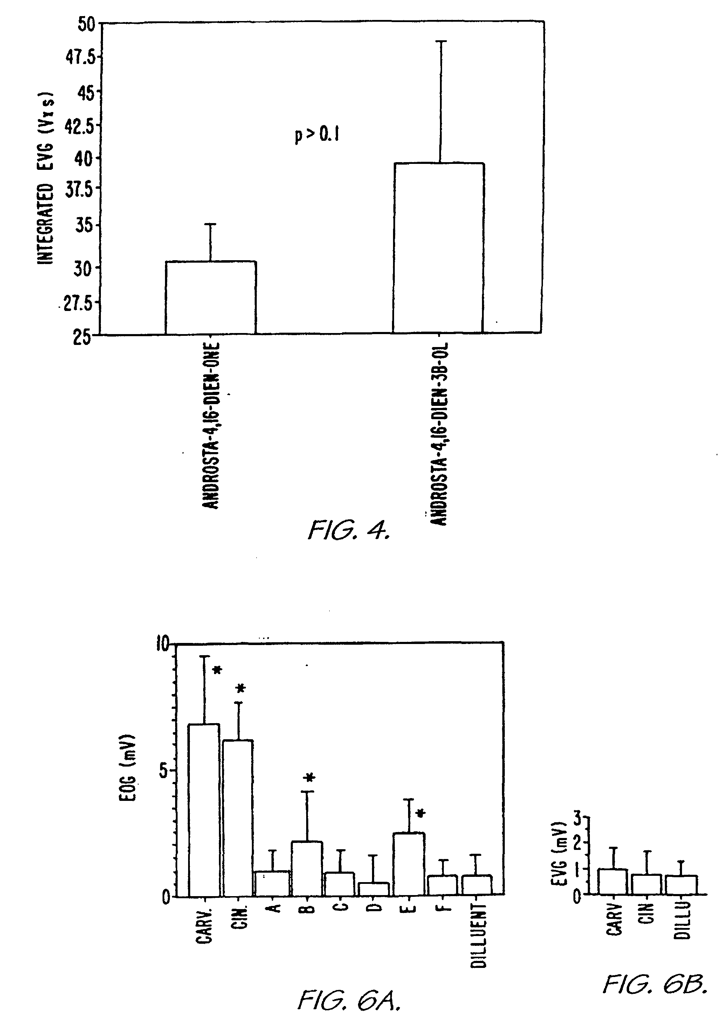

Cortical evoked activity was induced by VNO stimulation with vomeropherins, and olfactory stimulation with odorants delivered in 300 ms air pulses. It was recorded using standard electroencephalographic (EEG) electrodes placed at positions Cz-A1 and Tz-A1 of the international 10120 system; the ground electrode was placed on the mastoid process. Electrodermal activity (EDA) was recorded using standard 8 mm silver electrodes in contact with palmar skin of the medial and ring fingers respeCtively, through a conductive gel interface. Skin temperature (ST) was recorded by a small (1.0 mm) thermistor probe placed in the right ear lobe. Peripheral arterial pulse (PAP) was monitored with a plethysmograph attached to the tip of the index finger. Respiratory frequency (RF) was measured with an adjustable strain gauge placed around the lower thorax. All electrical signals were DC amplified, digitized (MP-100, Biopac Systems) and continuously monitored utilizing a computer. Statistical Analysis: EVGs or EOGS, peak-to-peak changes and frequency changes of other parameters were measured and statistically analyzed. The significance of the results was determined by either using paired t-tests or analysis of variance (ANOVA). Effect of Vomeropherins on the EVG: Each of the vomeropherins was found to produce a sexually dimorphic receptor potential (Fig. 5A-B). Recordings of the EVG were performed on 30 men and 30 women (ages 20 to 45). Vomeropherins were diluted and applied as 1 second pulses to the VNO with b minute intervals between pulses when questioned, the subjects were not able to "smell" or otherwise consciously detect any of the vomeropherins. This finding is in agreement with results previously reported (Monti-Bloch, L. and Grosser, B.l. (1991) "Effect of putative pheromones on the electrical activity of the human vomeronasal organ and olfactory epithelium," J. Steroid Biochem. Molec. Biol. 39:573-582.) which indicated that neither olfactory nor vomeropherin test stimuli delivered to the VNO elicit a perceptible sensation at the delivered concentration.

Fig. 5A shows the average response of male subjects (ages 20 to 38) to the diluent, and to equimolar quantities (100 fmoles) of five vomeropherins (A, B, C, D and F), and to E, a stereoisomer of F. The profile of the response to each of the substances was similar in all subjects regardless of age, and no significant differences were revealed either by t-tests or by analysis of variance. For example, A, C and D produced significant effects (M₁₅ = 11.4 mV, SD = 3.6 mV; M₇₆ = 6.4 mV, SD 2.5 mV, and M₈₄ = 15.1 mV, SD = 4.9mV; p<0.01), that were consistent in all individual cases. Other vomeropherins depolarized the VNO-receptors to a much lesser extent, but with consistent mean response amplitudes from individual to individual. Vomeropherins active in male subjects produced larger responses than the diluent (p<0.001). B, F and similar concentrations of olfactants induced significantly reduced responses in the male VNO (Fig. 5A and Fig. 6).

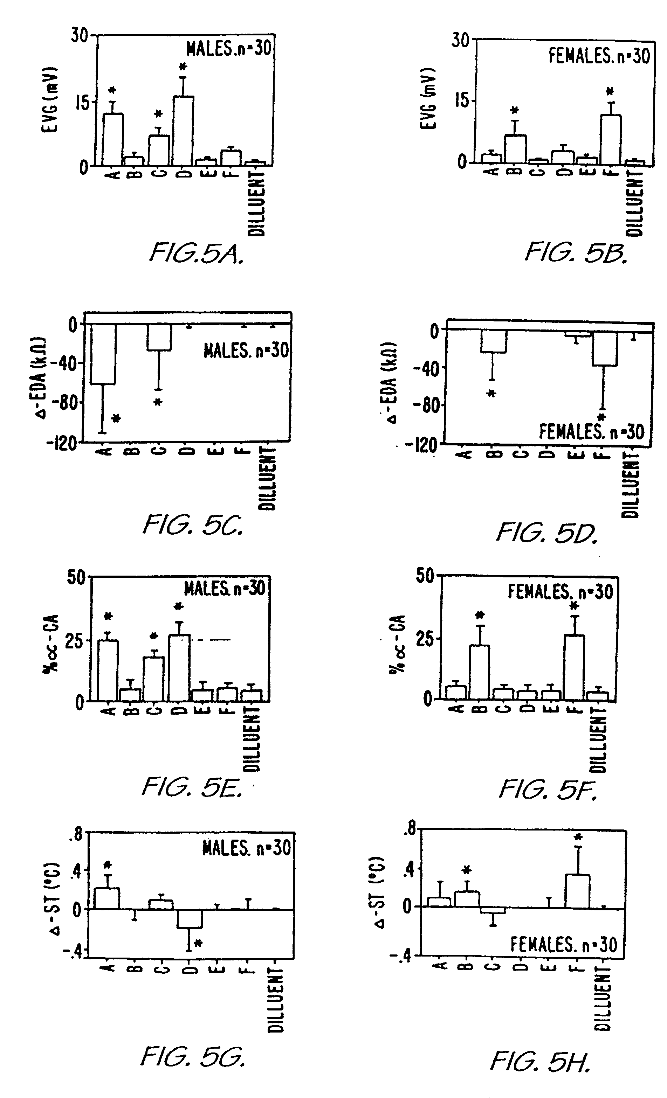

A similar experimental protocol was followed with the 30 female subjects (ages 20 - 45). Among the vomeropherins, F (100 fmoles) produced the most significant differences within the group (Fig. 5B). Here, A induced a small effect that was significantly different from F (p<0.01). In both populations of subjects, active vomeropherins induced receptor responses having large standard deviations (Fig. 5). When the frequency distribution of the effects of A and F was studied in males and females respectively, we found a bimodal distribution. The significance of this observation is being studied at this point.

E, a stereoisomer of F, does not stimulate the VNO in female subjects while F does (Fig. 5B). This is a demonstration of the specificity of VNO recognition of vomeropherins. In this regard it is interesting to note that while F is a superior vomeropherin, E generates a stronger olfactory effect than does F (Fig. 5B and Fig. 6).
Effects of Vomeropherins on the EOG: The summated receptor potential from the olfactory epithelium (OE) was recorded in 20 subjects: 10 males and 10 females. In contrast to the sensitivity of the VNO to vomeropherins, the OE is less sensitive to these substances. This is true for both males and females (Fig. 6A). The mean receptor potential amplitude ranged from 2.3 mV to 0.78 mV. In this study, B was the only vomeropherin having significant effect in the OE (p<0.02). Of the subjects questioned about odorant sensations following each stimulus presentation, 16 reported no olfactory sensation, while three males and one female described B as an unpleasant odor. This finding reveals that at the concentrations used in our study, most vomeropherins are not effective stimulants of the olfactory receptors, but do have a clear effect on vomeronasal receptors.
Effects of Olfactants on the EVG and EOG: In contrast to vomeropherins, the olfactants 1-carvone and cineole produce only a minor local response in the VNO (Fig. 6B). This was true for both men and women. As expected, these olfactants produced a strong response in both men and women (p<0.01) when locally applied to the OE (Fig. 6A). The diluent depolarized olfactory receptors to a lesser extent than cineole or 1-carvone (p<0.01), and it did not produce an olfactory sensation.
Reflex Effects of Vomeropherins: Studies were conducted to determine the central nervous system (CNS) reflex responses to vomeropherin stimulation of the VNO. The sexually dimorphic local responses induced by vomeropherins (Fig. 5A and B) were mirrored in the autonomic response of male & female subjects. In male subjects (Fig. 5C), A and C decreased skin resistance (electrodermal activity = EDA) (p<0.01, n = 30). In female subjects. (Fig. 5B), F and B produced greater decrease in EDA than A or C (p<0.01, n = 30).

Vomeropherins A and C induced a significant increase in skin temperature (ST) (Fig. 5G) in 30 male subjects (p<0.01); however D induced significant temperature decrease (p<0.01). In 30 female subjects (Fig. 5H) B and F evoked a significant increase in skin temperature (ST) (p<0.01) compared to A and C. In female subjects vomeropherins produced changes in EDA and ST with a greater standard deviation than in males.

Cortical activity was recorded from Cz and Tz in male and female subjects during application to the VNO of air pulses (300 ms to 1 sec) containing 200 fmoles of vomeropherin (Fig. 5G and H). In males (Fig. 5E) A, C and D significantly increased alpha cortical activity with a latency of 270-380 ms. D and A evoked the strongest effect (p< 0.01). Synchronization of the EEG was sustained for 1.5 to 2.7 minutes after application of a single pulse of active substance. In females (Fig. 5F), a single pulse (200 fmoles) of B or F applied to the VNO increased alpha cortical independent of the response of olfactory receptors. We found characteristic specificities in the response of the human VNO and the olfactory epithelium which suggests that they are independent functional systems with separate connections to the CNS (Brookover, C. (1914) The nervus terminalis in adult man. J. Comp. Neurol. 24:131-135.) There is also preliminary evidence that the EVG is not associated with trigeminal nociceptor endings since application of a local anesthetic (2% lidocaine) to the respiratory epithelium of the nasal septum neither blocks nor diminishes the EVG (Monti-Bloch, L. and Grosser, B.l. (1991) "Effect of putative pheromones on the electrical activity of the human vomeronasal organ and olfactory epithelium," J. Steroid Biochem, Molec. Biol. 39:573-582.), also, subjects failed to report sensations of pain as a consequence of any of the stimulation procedures.

VNO receptors are clearly more sensitive to vomeropherins than to any of the olfactants tested; the opposite is true for olfactory receptors. While the OE may have receptor sites for some vomeropherins, the response specificity of the VNO is clearly different.

Sexual differences were noted in the specificities and effects of two groups of vomeropherins, A, C and D; and B and F. This suggests a possible receptor-related sexual dimorphism. The findings suggest the activation of components of the autonomic nervous system in the adult human by vomeropherin stimulation of the VNO.

Furthermore, the results suggest that stimulation of the VNO with vomeropherins produces synchronization of the EEG (Fig. 5G and H) Thus, the evidence herein indicates that the vomeronasal system responds to a variety of chemosensory stimuli, and that some are able to induce reflex autonomic activity.

## Claims

1. A pharmaceutical composition suitable for nasal administration in an individual; said composition comprising a therapeutically effective amount of an androstane steroid selected from androsta-4,16-dien-3β-ol and 17-methylene-androst-4-en-3α-ol, and a pharmaceutically acceptable carrier.

2. A composition according to claim 1 wherein the amount of said androstane is at least 100 picograms but no more than 100 micrograms.

3. A composition according to claim 2 wherein the amount of said androstane is at least 1 nanogram but no more than 10 micrograms.

4. A composition according to claim 3 wherein the amount of said androstane is at least 10 nanograms but no more than 1 microgram.

5. A composition according to any of claims 1-4 wherein said androstane steroid is dissolved in said carrier.

6. A composition according to any of claims 1-4 wherein said composition is in liquid form.

7. A composition according to any of claims 1-4 wherein said composition further contains a pharmaceutically acceptable ointment base.

8. A composition according to any of claims 1-4 wherein said composition is formulated as an aerosol.

9. A composition according to any preceding claim for
(a) reducing premenstrual stress in women, or
(b) alleviating the symptoms of psychoses, depression or anxiety in an individual.

10. A composition according to claim 9 for alleviating the symptoms of psychosis, depression or anxiety.

11. A composition according to claim 10 for alleviating the symptoms of anxiety comprising androsta-4,16-dien-3β-ol.

12. A composition according to claim 10 for alleviating the symptoms of depression comprising 17-methylene-androst-4-en-3α-ol.

13. A composition according to any preceding claim containing more than one of said steroids.

14. Use of androsta-4,16-dien-3β-ol, or 17-methylene-androst-4-en-3α-ol in the preparation of a medicament suitable for nasal administration for
(a) reducing premenstrual stress in women, or
(b) alleviating the symptoms of psychoses, depression or anxiety in an individual.

15. Use according to claim 14 wherein said medicament comprises a therapeutically effective amount of said androstane of at least 100 picograms but no more than 100 micrograms.

16. Use according to claim 15 wherein the amount of said androstane is at least 100 picograms but no more than 100 micrograms.

17. Use according to claim16 wherein the amount of said androstane is at least 1 nanogram but no more than 10 micrograms.

18. Use according to any of claims 14-17 wherein said medicament is comprises in a pharmaceutical composition dissolved in a pharmaceutically acceptable carrier.

19. Use according to any of claims 14-17 wherein said pharmaceutical composition is an ointment.

20. Use according to any of claims 14-17 wherein said pharmaceutical composition is liquid.

21. Use according to any of claims 14-17 wherein said pharmaceutical composition is formulated as an aerosol.

22. Use according to any of claims 14 to 21 for reducing premenstrual stress in women

23. Use according to any of claims 14 to 21 for alleviating the symptoms of psychoses, depression or anxiety in an individual.

24. Use according to claim 23 for alleviating the symptoms of anxiety comprising androsta-4,16-dien-3β-ol.

25. Use according to claim 23 for alleviating the symptoms of depression comprising 17-methylene-androst-4-en-3α-ol.

26. Use according to any of claims 14 to 25 wherein the medicament contains more than one of said steroids.

27. The compound of the formula 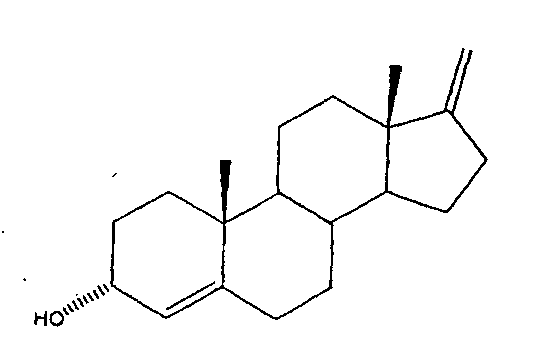 which is 17-methyleneandrost-4-en-3α-ol.

## Patentansprüche

1. Arzneimittelzusammensetzung, die für die nasale Verabreichung an ein Individuum geeignet ist, wobei die Zusammensetzung eine therapeutisch wirksame Menge eines Androstan-Steroids, das unter Androsta-4,16-dien-3β-ol und 17-Methylen-androst-4-en-3α-ol ausgewählt ist, und einen pharmazeutisch geeigneten Träger enthält.

2. Zusammensetzung nach Anspruch 1, wobei die Menge des Androstans mindestes 100 Picogramm, jedoch nicht mehr als 100 Mikrogramm, beträgt.

3. Zusammensetzung nach Anspruch 2, wobei die Menge des Androstans mindestes 1 Nanogramm, jedoch nicht mehr als 10 Mikrogramm beträgt.

4. Zusammensetzung nach Anspruch 3, wobei die Menge des Androstans mindestes 10 Nanogramm, jedoch nicht mehr als 1 Mikrogramm beträgt.

5. Zusammensetzung nach einem der Ansprüche 1 bis 4, wobei das Androstan-Steroid in dem Träger gelöst ist.

6. Zusammensetzung nach einem der Ansprüche 1 bis 4, wobei die Zusammensetzung in flüssiger Form vorliegt.

7. Zusammensetzung nach einem der Ansprüche 1 bis 4, wobei die Zusammensetzung weiterhin eine pharmazeutisch geeignete Salbengrundlage enthält.

8. Zusammensetzung nach einem der Ansprüche 1 bis 4, wobei die Zusammensetzung als Aerosol zubereitet ist.

9. Zusammensetzung nach einem der vorhergehenden Ansprüche zum
(a) Vermindern des prämenstruellen Stress bei Frauen oder
(b) Erleichtern der Symptome von Psychosen, Depression oder Ängstlichkeit bei einem Individuum.

10. Zusammensetzung nach Anspruch 9 zum Erleichtern der Symptome einer Psychose, von Depression oder Ängstlichkeit.

11. Zusammensetzung nach Anspruch 10 zum Erleichtern der Symptome von Ängstlichkeit, die Androsta-4,16-dien-3β-ol enthält.

12. Zusammensetzung nach Anspruch 10 zum Erleichtern der Symptome einer Depression, die 17-Methylen-androst-4-en-3 α-ol enthält.

13. Zusammensetzung nach einem vorhergehenden Anspruch, die mehr als eines dieser Steroide enthält.

14. Verwendung von Androsta-4,16-dien-3β-ol oder 17-Methylen-androst-4-en-3α-ol bei der Herstellung eines Arzneimittels, das sich für die nasale Verabreichung zum
(a) Vermindern des prämenstruellen Stress bei Frauen oder
(b) Erleichtern der Symptome von Psychosen, Depression oder Ängstlichkeit bei einem Individuum eignet.

15. Verwendung nach Anspruch 14, wobei das Arzneimittel eine therapeutisch wirksame Menge des Androstans von mindestens 100 Picogramm, jedoch nicht mehr als 100 Mikrogramm, enthält.

16. Verwendung nach Anspruch 15, wobei die Menge des Androstans mindestens 100 Picogramm, jedoch nicht mehr als 100 Mikrogramm beträgt.

17. Verwendung nach Anspruch 16, wobei die Menge des Androstans mindestens 1 Nanogramm, jedoch nicht mehr als 10 Mikrogramm beträgt.

18. Verwendung nach einem der Ansprüche 14 bis 17, wobei das Arzneimittel in einer Arzneimittelzusammensetzung, gelöst in einem pharmazeutisch geeigneten Träger, vorliegt.

19. Verwendung nach einem der Ansprüche 14 bis 17, wobei die Arzneimittelzusammensetzung eine Salbe ist.

20. Verwendung nach einem der Ansprüche 14 bis 17, wobei die Arzneimittelzusammensetzung flüssig ist.

21. Verwendung nach einem der Ansprüche 14 bis 17, wobei die Arzneimittelzusammensetzung als Aerosol zubereitet ist.

22. Verwendung nach einem der Ansprüche 14 bis 21 zum Vermindern des prämenstruellen Stress bei Frauen.

23. Verwendung nach einem der Ansprüche 14 bis 21 zum Erleichtern der Symptome von Psychosen, Depression oder Ängstlichkeit bei einem Individuum.

24. Verwendung nach Anspruch 23 zum Erleichtern der Symptome der Ängstlichkeit, wobei Androsta-4,16-dien-3β-ol enthalten ist.

25. Verwendung nach Anspruch 23 zum Erleichtern der Symptome von Depression, wobei 17-Methylen-androst-4-en-3α-ol enthalten ist.

26. Verwendung nach einem der Ansprüche 14 bis 25, wobei das Arzneimittel mehr als eines dieser Steroide enthält.

27. Verbindung der Formel 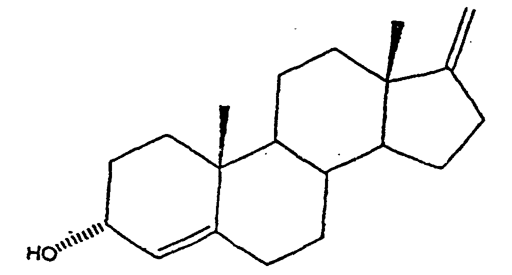 die 17-Methylenandrost-4-en-3α-ol ist.

## Revendications

1. Composition pharmaceutique apte à l'administration nasale chez un individu ; ladite composition comprenant une quantité thérapeutiquement efficace d'un stéroide du type androstane, choisi entre l'androsta 4, 16-dien-3β-ol et le 17-méthylène-androst-4-en-3α-ol, et un support pharmaceutiquement acceptable.

2. Composition suivant la revendication 1, dans laquelle la quantité dudit androstane est égale à au moins 100 picrogrammes mais non supérieure à 100 microgrammes.

3. Composition suivant la revendication 2, dans laquelle la quantité dudit androstane est égale à au moins 1 nanogramme mais non supérieure à 10 microgrammes.

4. Composition suivant la revendication 3, dans laquelle la quantité dudit androstane est égale à au moins 10 nanogrammes mais non supérieure à 1 microgramme.

5. Composition suivant l'une quelconque des revendications 1 à 4, dans laquelle ledit stéroïde consistant en androstane est dissout dans ledit support.

6. Composition suivant l'une quelconque des revendications 1 à 4, qui est sous forme liquide.

7. Composition suivant l'une quelconque des revendications 1 à 4, qui contient en outre un excipient pour pommade pharmaceutiquement acceptable.

8. Composition suivant l'une quelconque des revendications 1 à 4, qui est formulée à l'état d'aérosol.

9. Composition suivant l'une quelconque des revendications précédentes, pour
a) réduire le stress prémenstruel chez la femme,
b) soulager les symptômes de la psychose, de la dépression et de l'anxiété chez un individu.

10. Composition suivant la revendication 9, destinée à soulager les symptômes de la psychose, de la dépression et de l'anxiété.

11. Composition suivant la revendication 10, destinée à soulager les symptômes de l'anxiété, comprenant de l'andosta-4, 16-dien-3β-ol.

12. Composition suivant la revendication 10, destinée à soulager les symptômes de la dépression, comprenant du 17-méthylène-andost-4-en-3α-ol.

13. Composition suivant l'une quelconque des revendications précédentes, contenant plus d'un desdits stéroïdes.

14. Utilisation de l'androsta-4, 16-dien-3β-ol ou du 17-méthylène-androst-4-3α-ol dans la préparation d'un médicament apte à l'administration nasale pour
a) réduire le stress prémenstruel chez la femme, ou
b) soulager les symptômes de psychose, de la, dépression ou de l'anxiété chez un individu.

15. Utilisation suivant la revendication 14, dans laquelle ledit médicament comprend une quantité thérapeutiquement efficace dudit androstane égale à au moins 100 picogrammes mais non inférieure à 100 microgrammes.

16. Utilisation suivant la revendication 15, dans laquelle la quantité dudit androstane est égale à au moins 100 picrogrammes mais non supérieure à 100 microgrammes.

17. Utilisation suivant la revendication 16, dans laquelle la quantité dudit androstane est égale à au moins 1 nanogramme mais non supérieure à 10 microgrammes.

18. Utilisation suivant l'une quelconque des revendications 14 à 17, dans laquelle ledit médicament est présent dans une composition pharmaceutique à l'état dissout dans un support pharmaceutiquement acceptable.

19. Utilisation suivant l'une quelconque des revendications 14 à 17, dans laquelle ladite composition pharmaceutique est une pommade.

20. Utilisation suivant l'une quelconque des revendications 14 à 17, dans laquelle ladite composition pharmaceutique est liquide.

21. Utilisation suivant l'une quelconque des revendications 14 à 17, dans laquelle ladite composition pharmaceutique est formulée à l'état d'aérosol.

22. Utilisation suivant l'une quelconque des revendications 14 à 21, pour réduire le stress prémenstruel chez la femme.

23. Utilisation suivant l'une quelconque des revendications 14 à 21, pour soulager les symptômes de psychose, de la dépression ou de l'anxiété chez un individu.

24. Utilisation suivant la revendication 23, pour soulager les symptômes de l'anxiété, comprehant de l'androsta-4, 16-dien-3β-ol.

25. Utilisation suivant la revendication 23, pour soulager les symptômes de la dépression, comprenant du 17-méthylène-androst-4-en-3α-ol.

26. Utilisation suivant l'une quelconque des revendications 14 à 25, dans laquelle le médicament contient plus d'un desdits stéroïdes.

27. Composés de formule 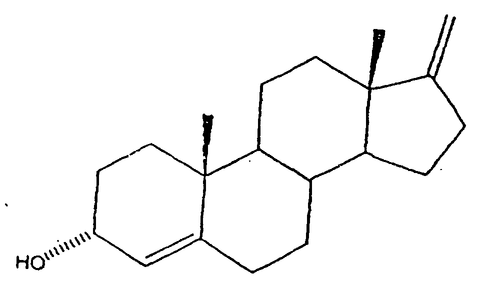 qui est le 17-méthylène-androst-4-en-3α-ol.
